# DEMANDE DE BREVET EUROPEEN

(11) **EP 1 318 203 A2**
(43) Date de publication de la demande: **11.06.2003**
(21) Numéro de dépôt: 02022482.0
(22) Date de dépôt: 26.07.1994
(51) Int. Cl.: C12Q 1/68

(54) **Procédé d'amplification d'acides nucléiques par élongation et déplacement**

(30) Priorité: 26.07.1993 FR 9309187
(62) Demande divisionnaire de: 94922306.9
(71) Demandeur: BIOMERIEUX, 69280 Marcy-L'Etoile (FR)
(72) Inventeur: Cleuziat, Philippe, 69003 Lyon (FR); Guillou-Bonnici, Francoise, 69100 Villeurbanne (FR); Mallet, Francois, 69100 Villeurbanne (FR); Asseur, Pierre, 69002 Lyon (FR)
(74) Mandataire: Tonnellier, Jean-Claude

(57) **Abrégé**

Méthode pour générer un polynucléotide double brin contenant une séquence complémentaire d'une séquence d'intérêt et un polynucléotide simple brin contenant une séquence complémentaire de ladite séquence d'intérêt, ladite méthode comprenant les étapes consistant :
- à obtenir un acide nucléique simple brin comprenant ladite séquence d'intérêt, et
- à mettre en contact ledit acide nucléique simple brin avec une première et une seconde amorces, en présence d'une activité de polymérase et d'une activité de déplacement de brin, ladite première amorce s'hybridant à une première séquence dudit acide nucléique simple brin et ladite seconde amorce s'hybridant à une seconde séquence dudit acide nucléique simple brin, ladite seconde séquence chevauchant ladite première séquence, et lesdites première et seconde séquences étant situées en aval de l'extrémité 3' de ladite séquence d'intérêt, de sorte que le produit d'élongation de l'une des amorces déplace le produit d'élongation de l'autre amorce, grâce à quoi on obtient un polynucléotide double brin contenant une séquence complémentaire de ladite séquence d'intérêt et un polynucléotide simple brin déplacé contenant une séquence complémentaire de ladite séquence d'intérêt.

## Description

La présente invention concerne une méthode pour l'amplification de séquences d'acide nucléique cibles. En particulier, la présente invention consiste en une méthode pour l'amplification d'acides nucléiques, utilisant l'élongation et le déplacement.

Il est souvent nécessaire, dans les technologies relatives aux acides nucléiques et au matériel génétique, de déterminer si un gène, une partie de gène ou une séquence nucléotidique est présent chez un organisme vivant, un extrait cellulaire de cet organisme ou un échantillon biologique. Puisque tout gène ou partie de gène est une séquence spécifique de bases nucléotidiques formant tout ou partie d'une molécule nucléotidique, il est possible de rechercher directement la présence d'une séquence nucléotidique spécifique au sein d'un échantillon contenant des polynucléotides.

L'intérêt de la recherche de séquences nucléotidiques spécifiques est immense, notamment pour la détection d'organismes pathogènes, la détermination de la présence d'allèles, la détection de la présence de lésions dans un génome hôte et la détection de la présence d'un ARNm particulier ou de la modification d'un hôte cellulaire. Les maladies génétiques telles que la maladie de Huntington, la myopathie de Duchenne, la phénylcétonurie et la 13-thalassémie peuvent être diagnostiquées par le biais de l'analyse de l'ADN des individus. De plus, le diagnostic ou l'identification des virus, viroïdes, bactéries, champignons, protozoaires ou quelque autre forme de vie végétale ou animale peut être réalisé par des expériences d'hybridation avec des sondes nucléiques.

Différents types de méthodes de détection des acides nucléiques sont décrits dans la littérature. Ces méthodes, et particulièrement celles qui requièrent la détection de polynucléotides, reposent sur les propriétés d'appariement purine-pyrimidine des brins complémentaires d'acides nucléiques dans les duplex ADN-ADN, ADN-ARN et ARN-ARN. Ce processus d'appariement s'effectue par l'établissement de liaisons hydrogène entre les bases adénosine-thymine (A-T) et guanosine-cytosine (G-C) de l'ADN double brin ; des paires de bases adénosine-uracile (A-U) peuvent également se former par liaison hydrogène dans les duplex ADN-ARN ou ARN-ARN. L'appariement de brins d'acides nucléiques pour la détermination de la présence ou de l'absence d'une molécule d'acide nucléique donnée est communément appelée "hybridation d'acides nucléiques" ou simplement "hybridation".

Dans les quelques exemples cités précédemment, après avoir identifié une séquence spécifique d'un organisme ou d'une maladie, il convient d'extraire les acides nucléiques d'un échantillon, et de déterminer si cette séquence est présente. De nombreuses méthodes de détection ont été développées dans ce but.

S'il est capital qu'une ou plusieurs séquences spécifiques d'une maladie ou d'un organisme soient identifiées, la nature de ces séquences et la façon dont elles ont été identifiées n'ont aucune importance particulière pour la mise en oeuvre de la présente invention. La méthode la plus directe pour détecter la présence d'une séquence cible dans un échantillon d'acides nucléiques est d'obtenir une "sonde" dont la séquence est suffisamment complémentaire d'une partie de l'acide nucléique cible pour s'hybrider à celui-ci. La sonde ainsi synthétisée peut être appliquée dans un échantillon contenant des acides nucléiques, et si la séquence cible est présente, la sonde s'hybridera pour former un produit de réaction. En l'absence de séquence cible et en évitant tout phénomène d'hybridation non spécifique, aucun produit de réaction ne sera formé. Si la sonde synthétisée est couplée à un marqueur détectable, le produit de réaction peut être détecté en mesurant la quantité de marqueur présent. Le transfert de Southern (Southern E.M., *J. Mol. Biol.*, 98, 503 (1975)) ou l'hybridation sandwich (Dunn A.R., Hassel *J. A.*, *Cell,* 12, 23, (1977)) constituent des exemples où ces méthodes sont utilisées.

La principale difficulté de cette approche est, cependant, qu'elle n'est pas directement applicable aux cas où le nombre de copies de la séquence cible présente dans un échantillon est faible (c'est à dire inférieur à 10⁷). Dans ces conditions, il est difficile de distinguer un signal significatif, c'est à dire supérieur au bruit de fond de réaction (c'est à dire de distinguer la fixation spécifique d'une sonde sur sa séquence cible de la fixation non spécifique entre la sonde et une séquence différente de la séquence cible). Une des solutions à ce problème consiste à augmenter le signal de détection par une réaction supplémentaire. En conséquence, diverses méthodes ont été décrites afin d'accroître la puissance de détection de ces techniques d'hybridation. Ces méthodes dites "d'amplification" peuvent intervenir à différents stades d'un procédé de détection par sondes nucléiques. Ces stades peuvent être classés en trois catégories : l'amplification de cible, de sonde ou de signal. Les articles de Lewis (1992. *Genetic Engineering News* 12 : 1-9) d'une part, et d'Abramson et Myers (1993. *Curr. Opin. Biotechnol*. 4 : 41-47), d'autre part, constituent de bonnes revues générales de ces méthodes.

L'amplification de cible consiste à multiplier de manière spécifique un fragment d'acide nucléique présent dans un échantillon. Elle permet d'augmenter considérablement le nombre de copies d'une séquence nucléique cible à détecter.

La méthode la plus connue est la "Polymerase Chain Reaction" (dite PCR), technique d'amplification de cible qui repose sur la répétition de cycles de synthèse d'ADN *in vitro* par élongation d'amorces nucléotidiques hybridées sur la séquence cible (Saiki *et al.*, 1985. *Science* 230 : 1350-1354 ; brevets des États Unis d'Amérique n° 4 683 195, 4 683 202 et 4 800 159 ; brevet Européen n° 0 201 184). Brièvement, deux amorces nucléotidiques complémentaires chacune d'une séquence d'un des deux brins de l'ADN cible sont synthétisées. Des désoxyribonucléosides triphosphates sont ajoutés en excès au milieu réactionnel en présence d'une ADN polymérase ADN-dépendante thermostable (*Taq* polymérase). Si l'ADN cible est présent dans l'échantillon, les amorces s'hybrident sur leurs sites spécifiques et la polymérase étend l'extrémité 3' de ces amorces par addition successive des nucléotides complémentaires de la cible. En réalisant des cycles successifs de montée et descente de température, les amorces étendues se dissocient de la cible et peuvent, comme la cible originale, fixer les amorces nucléotidiques en excès. Par répétition du processus (de 20 à 40 fois), on aboutit à une accumulation exponentielle de la séquence cible comprise entre les deux amorces.

Une autre méthode utilisant des cycles de température est décrite dans le brevet Européen n° 0 320 308 et est appelée "Ligase Chain Reaction" (dite LCR). Deux sondes oligonucléotidiques adjacentes, ainsi que deux autres qui leurs sont complémentaires sont mises en excès dans le milieu réactionnel, en présence d'une ADN Ligase. En présence de l'ADN cible, chaque oligonucléotide s'hybride sur sa séquence complémentaire et la ligase peut lier les deux sondes hybridées de manière adjacente. Par succession de cycles de température, comme dans la PCR, et par l'utilisation d'une ligase thermostable (Barany, 1991. *Proc. Natl. Acad. Sci.* USA 88 : 189-193), les sondes liées se dissocient de la cible et peuvent à leur tour servir de séquence cible pour les sondes en excès.

La méthode "Repair Chain Reaction" (dite RCR) est une méthode d'amplification voisine de la LCR (demande de brevet n° WO 90/01069). C'est une méthode hybride entre la PCR et la LCR. Elle utilise deux sondes oligonucléotidiques complémentaires de la cible et deux amorces en excès dans le milieu réactionnel, en présence d'une ADN ligase thermostable et d'une ADN polymérase thermostable. En présence d'ADN cible, les oligonucléotides s'hybrident sur leur séquence cible et un espace de quelques bases sépare l'extrémité de chaque amorce de la sonde oligonucléotidique adjacente. La polymérase comble cet espace et rend aussi possible l'action de la ligase qui lie l'amorce élongée à la sonde adjacente, mimant ainsi les processus naturels de réparation de l'ADN. Par succession de cycles de température, comme dans la PCR et la LCR, les amorces élongées liées aux sondes oligonucléotiques peuvent à leur tour servir de cible pour les amorces et les sondes en excès.

Il existe d'autres méthodes d'amplification de cible basées sur l'amplification de transcrits (dite TAS). La TAS, décrite dans la demande de brevet n° WO 88/10315, consiste en la répétition d'un cycle de trois étapes. La première étape permet de synthétiser un ADNc à partir d'ARN en présence de transcriptase inverse et d'une amorce désoxynucléotidique "hybride" contenant une séquence spécifique de promoteur d'ARN polymérase phagique. Suite à la dénaturation thermique de l'hétéroduplex ARN/ADNc, le brin monocaténaire d'ADNc est répliqué par la transcriptase inverse en présence d'une amorce oligonucléotidique anti-sens. L'homoduplex d'ADN ainsi obtenu lors de cette deuxième étape contient un promoteur double brin sur lequel une ARN polymérase ADN-dépendante phagique peut se fixer. La troisième étape consiste alors à transcrire des molécules d'ARN (de 30 à 1000 par matrice) qui pourront de nouveau servir de matrice pour la synthèse d'ADNc et poursuivre ainsi le cycle d'amplification (Davis *et al.*, 1990. *J. Infect. Dis*. 162 : 13-20).

Il existe différentes méthodes dérivées de la TAS, dont la "Self-Sustained Sequence Replication" (dite 3SR), décrite dans la demande de brevet WO 90/06995 et le brevet Européen n° 0 373 960, la "Nucleic Acid Sequence-Based Amplification" (dite NASBA) décrite dans la demande de brevet WO 91/02818 et le brevet Européen n° 0 329 822 et la "Single Primer Sequence Replication" (dite SPSR) décrite dans le brevet des États Unis d'Amérique n° 5 194 370. Ces méthodes possèdent en commun l'association de trois activités enzymatiques: ADN polymérase ARN- et ADN-dépendante (transcriptase inverse), ribonucléase H (RNase H ; enzyme d'Escherichia coli et/ou activité enzymatique associée de la transcriptase inverse), et ARN polymérase ADN-dépendante (ARN polymérase du bactériophage T7). Ces méthodes sont basées sur le même principe et s'effectuent à une température fixe (de 37 à 45°C), selon un processus continu de réactions de transcription inverse et de transcription afin de répliquer une cible d'ARN par l'intermédiaire d'ADNc. Comme dans le cas de la TAS, un site de fixation d'une ARN polymérase (phage T7) est introduit dans l'ADNc par l'amorce utilisée pour l'étape de transcription inverse. Néanmoins, la dénaturation de l'hétéroduplex ARN/ADNc est effectuée de façon isotherme par hydrolyse spécifique de l'ARN de cet hétéroduplex par l'activité RNase H. L'ADNc libre est alors répliqué à partir d'une seconde amorce oligonucléotidique par la transcriptase inverse. L'homoduplex ADN/ADN est transcrit en ARN par l'ARN polymérase T7 et cet ARN peut à nouveau servir de matrice pour le cycle suivant.

Une autre méthode, la "Ligation Activated Transcription" (dite LAT) décrite dans le brevet des États Unis d'Amérique n° 5 194 370, utilise les mêmes activités enzymatiques que la 3SR, SPSR et NASBA et fonctionne sur le même cycle. Elle diffère cependant par le mode d'installation d'une séquence promotrice qui dans ce cas est introduite sur l'extrémité de l'ADNc par ligation d'une structure tige-boucle contenant le promoteur, en présence d'une ADN ligase.

D'autres méthodes d'amplification de cible sont actuellement décrites. La méthode "Strand Displacement Amplification" (dite SDA), décrite dans le brevet Européen n° 0 497 272, permet une multiplication isotherme (37°C) d'une séquence d'ADN cible à l'aide d'une enzyme de restriction appropriée et d'une ADN polymérase ADN-dépendante dépourvue d'activité exonucléase (Walker *et al.*, 1992. *Proc. Natl. Acad. Sci. USA* 89 : 392-396). Elle est basée sur l'hybridation d'amorces oligonucléotidiques contenant une séquence de reconnaissance d'une enzyme de restriction à leur extrémité 5'. Ces amorces sont élongées sur la cible par l'ADN polymérase en présence d'au moins un nucléotide modifié (5'[α-thio]dNTP). La digestion de l'ADN par l'endonucléase de restriction provoque la coupure du brin correspondant à l'amorce, laissant intact le brin complémentaire modifié. L'ADN polymérase peut réaliser l'élongation de l'amorce ainsi générée et libère un brin d'ADN dans le milieu réactionnel grâce à la propriété de déplacement de brin de l'ADN polymérase. Ce brin libéré peut alors fixer une amorce contenant une séquence de fixation d'enzyme de restriction et le cycle peut alors recommencer. Par ailleurs, il est décrit dans la demande de brevet européen 543 612 une méthode de préparation d'acides nucléiques présentant des extrémités définies et pouvant servir ultérieurement dans une méthode d'amplification, notamment la SDA précitée. Il convient toutefois de noter que cette méthode de préparation, en elle même, ne peut en aucun cas donner lieu à un cycle d'amplification isotherme. Une méthode similaire à la SDA utilisant une activité exonucléase au lieu de endonucléase, appelée "exonuclease-mediated strand displacement amplification", est décrite dans le brevet Européen n° 0 500 224.

Pour toutes les méthodes décrites précédemment, une variété de méthodes de détection peut être utilisée. L'une d'entre elles consiste à détecter les produits de réaction ayant une taille définie par séparation électrophorétique. Les méthodes varient selon le procédé de séparation, qui peut impliquer la séparation sur gel, la fixation sur différentes phases solides (billes, plaque de microtitrage, latex, particules magnétiques). Une autre méthode utilise le marquage d'une sonde de détection avec un radioisotope tel que le ³² P, par exemple, puis la détection de la radioactivité émise par les produits de réaction en combinaison ou non avec une électrophorèse. Une autre méthode consiste à modifier chimiquement une amorce oligonucléotidique en y ajoutant un ligand (la biotine ou la digoxigénine, par exemple), une enzyme (la phosphatase alcaline, la peroxydase, la β-galactosidase, par exemple), un marqueur fluorescent (la phycobiliprotéine, la fluorescéine ou la rhodamine, par exemple), un marqueur luminescent (un ester d'acridinium, par exemple) ou une combinaison de ces modifications. Une autre méthode consiste à développer une amorce nucléotidique de détection qui s'hybridera sur le produit de réaction d'amplification et sera élongée par une polymérase en présence de ribonucléosides triphosphates (cette amorce peut dans ce cas être également modifiée comme décrit précédemment). Toutes ces méthodes peuvent être adaptées sur phase solide comme en solution (phase homogène).

Néanmoins, toutes les techniques d'amplification précédemment présentées possèdent au moins une limitation importante. Elles ne permettent d'obtenir un produit d'amplification qu'à partir d'un seul type d'acide nucléique cible : ARN ou ADN. Dans certains cas tels que la PCR, la LCR ou la RCR, le facteur le plus limitant est la nécessité de réaliser de nombreux cycles de températures afin de dissocier les produits de réaction de la cible. Ceci limite le choix des enzymes utilisables aux enzymes thermostables. De plus, la réalisation de tels cycles successifs de température constitue un inconvénient pour l'automatisation de ces techniques. Un autre inconvénient de certaines techniques d'amplification est la limitation de la taille du produit de réaction d'amplification. Les techniques telles que la RCR ou la LCR ne permettent d'amplifier que la séquence de la cible correspondant aux amorces et aux sondes nucléotidiques utilisées dans le processus d'amplification. Le bruit de fond non spécifique (c'est à dire en absence de la cible) est également un sérieux inconvénient de certaines techniques, telle que la LCR ; dans le cas de la LCR, une ligation des extrémités des oligonucléotides libres en excès s'effectue en absence de la cible. D'autres méthodes telles que la SDA sont limitées dans le type de séquence cible à amplifier, puisque cette séquence ne doit pas comporter de site de restriction correspondant à l'endonucléase utilisée dans le procédé, il est donc indispensable de connaître sinon la séquence nucléique du fragment à amplifier au moins la carte de restriction dudit fragment. En outre, cette limitation est accrue par le fait que le choix des endonucléases de restriction est restreint à celles ayant la capacité de cliver un site de reconnaissance hémiphosphorothioate (c'est à dire comprenant un brin d'ADN du duplex avec au moins un nucléotide modifié de type phosphorothioate), et, sur un plan plus général, les sites comportant des nucléotides modifiés. Outre les limitations du choix du nucléotide modifié, dues à la synthèse chimique, le procédé d'amplification est également limité par son rendement puisqu'il est connu que le Km des polymérases pour les nucléotides modifiés est supérieur à celui pour les nucléotides naturels pour les polymérases, d'où une plus faible efficacité d'incorporation enzymatique des nucléotides modifiés dans la cible à amplifier. Un autre inconvénient majeur de certaines techniques d'amplification réside dans le nombre élevé d'activités enzymatiques impliquées dans le processus d'amplification. Les méthodes dérivées de la TAS, telle que la 3SR ou la NASBA requièrent au moins quatre activités enzymatiques (ADN polymérase ADN-dépendante, ADN polymérase ARN-dépendante, ARN polymérase ADN dépendante, RNase H), voire cinq dans le cas de la LAT (ADN ligase en plus). Il est par conséquent très difficile de rendre ces techniques performantes du fait de la difficulté de parvenir à des conditions réactionnelles satisfaisant simultanément ces quatre ou cinq activités enzymatiques. De plus, ces techniques transcriptionnelles ne permettent une amplification qu'à partir de molécules cibles ARN, mais pas ADN. Enfin, si diverses techniques d'amplification ont recours à des activités nucléases (exonucléase, endonucléase, RNase) comme moyen de séparation de brins d'acides nucléiques (brevet Européen n° 0 500 224), leur utilisation est néanmoins préjudiciable, du fait de la possibilité de dégradation de la cible ou du produit de réaction par des activités nucléases parasites parfois présentes dans des préparations de telles activités nucléases. De plus, dans le cas des techniques transcriptionnelles, l'utilisation de RNases conduit à une dégradation partielle du matériel amplifié, et nuit donc au rendement et à la sensibilité de la technique. De plus, l'action de la RNase doit être rigoureusement dosée, afin de maintenir un équilibre avec l'action des différentes enzymes impliquées. Au vu de ces nombreuses limitations, d'autres méthodes d'amplification alternatives à ces méthodes existantes sont souhaitables.

Selon un premier aspect, l'invention concerne une méthode d'amplification d'une séquence d'acide nucléique cible (ARN et/ou ADN) de séquence quelconque (ainsi que sa séquence complémentaire) dans un échantillon, par réaction de transcription utilisant le déplacement. La méthode inclut différentes étapes permettant la mise en évidence d'une séquence d'acide nucléique cible dans un échantillon. La méthode peut comprendre notamment : 1) l'extraction des acides nucléiques d'un échantillon biologique susceptible de contenir la séquence cible recherchée, 2) la dénaturation de la séquence cible, c'est à dire l'obtention de fragments simple brin si la cible est un duplex, 3) l'addition d'un mélange réactionnel contenant (a) une enzyme ou un mélange d'enzymes ayant une activité ADN polymérase ADN- et ARN-dépendante et une activité de déplacement de brin, (b) une ARN polymérase, (c) une amorce oligonucléotidique composée successivement (de 5' vers 3') d'une séquence facultative arbitraire d'au moins 5 nucléotides, d'une séquence comportant tout ou partie d'une séquence promotrice d'une ARN polymérase, puis d'une séquence complémentaire de l'extrémité 3' d'un fragment d'acide nucléique cible, et/ou (d) une amorce oligonucléotidique facultative possédant à son extrémité 3' tout ou partie de la séquence arbitraire de l'amorce définie en 3c, et/ou (e) une amorce nucléotidique facultative possédant à son extrémité 3' tout ou partie de l'amorce définie en 3c, dont l'extrémité 3' s'hybride en aval (3') de l'amorce définie en 3d et pouvant comprendre au moins la partie 5' d'une séquence sens du promoteur mais ne comprenant pas l'extrémité 3' de l'amorce définie en 3c, (f) des désoxyribonucléosides triphosphates et des ribonucléosides triphosphates, (g) un tampon approprié pour le fonctionnement des activités enzymatiques et 4) la réalisation d'une incubation pendant une durée minimum afin de générer les produits de réaction.

La séparation et/ou la détection des produits de réaction peut être mise en oeuvre par diverses méthodes, dont celles décrites précédemment. Les méthodes de séparation comprennent, entre autres, la séparation magnétique, la capture sur support solide, sur membrane, sur filtre ou sur un polymère. Dans chaque méthode, un résidu de capture peut être fixé à une bille magnétique, à une membrane, à un filtre ou à un polymère. Les billes, la membrane, le support solide, le filtre ou le polymère peuvent être ensuite testés quant à la présence ou l'absence du produit de réaction de la méthode d'amplification. Les résidus de capture peuvent être, par exemple, une séquence d'acide nucléique complémentaire du produit de réaction d'amplification, des protéines ou des anticorps dirigés contre un ligand ou un haptène incorporé dans une des amorces utilisées, ou dans le produit de réaction. Le système de séparation peut être ou non couplé au système de détection.

Les systèmes de détection utiles pour la pratique de l'invention comprennent les systèmes homogènes (c'est à dire qui ne nécessitent pas de système de séparation) et, par opposition, les systèmes hétérogènes. Dans chaque système, un ou plusieurs marqueurs détectables sont utilisés et la réaction ou l'émission du système de détection est mesurée, de manière préférentielle par des moyens automatisés. Les exemples de systèmes de détection homogène comprennent le transfert d'énergie de fluorescence, la protection par hybridation (luminescence d'acridinium), la polarisation de fluorescence et la détection immunologique d'un donneur d'enzyme clonée. Les exemples de systèmes hétérogènes comprennent les marqueurs enzymatiques (peroxydase, phosphatase, β-galactosidase), les marqueurs fluorescents (marqueurs enzymatiques, rhodamine, fluorescéine), la chimiluminescence et la bioluminescence. Dans ces systèmes de détection, les marqueurs détectables peuvent être conjugués directement ou indirectement à un résidu de capture, ou les produits de réaction d'amplification peuvent être générés en présence d'une protéine ou d'un anticorps qui peut être reconnu par un ligand ou un haptène pour ces derniers.

On peut effectuer la détection indirecte d'une molécule d'intérêt par une méthode dans laquelle le polynucléotide qui est un marqueur couplé à la molécule d'intérêt est amplifié. Les produits d'amplification du polynucléotide marqueur peuvent eux-mêmes être détectés directement par l'incorporation lors de leur synthèse de nucléotides modifiés tels que marqués au [³² P] ou au [³ H] ou, peuvent être également détectés de façon indirecte selon les méthodes décrites précédemment.

L'invention concerne également les méthodes pour générer des produits d'amplification utilisables comme sonde, ou utilisables comme matrice pour la détermination de sa séquence nucléotidique. Les produits amplifiés peuvent être séparés des enzymes utilisées pour l'amplification (ADN polymérase et ARN polymérase), afin d'être utilisés dans des procédés ultérieurs impliquant d'autres réactions enzymatiques, d'autres systèmes d'amplification, des méthodes de séquençage ou des méthodes de synthèse d'acides nucléiques, pour ne citer que quelques exemples.

Un nécessaire pour la détection d'un acide nucléique cible susceptible d'être présent dans un échantillon peut être utilisé pour la mise en oeuvre de la méthode d'amplification décrite précédemment.

La présente invention a en particulier pour objet une méthode telle que définie dans les revendications ci-annexées. On va maintenant décrire l'invention de façon plus détaillée, et en faisant le cas échéant référence aux dessins annexés dans lesquels :

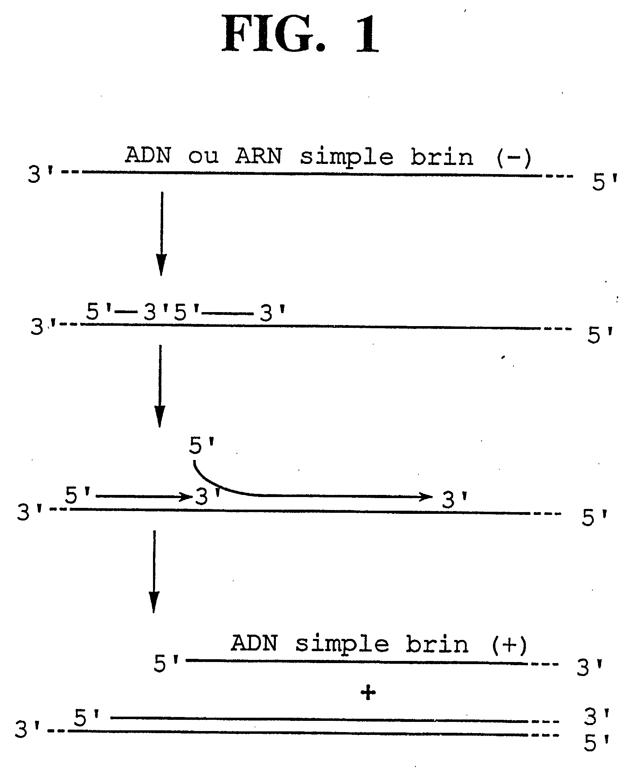

La figure 1 décrit le principe de séparation d'un brin d'acide nucléique d'un duplex par utilisation des propriétés de déplacement de brin de certaines polymérases.

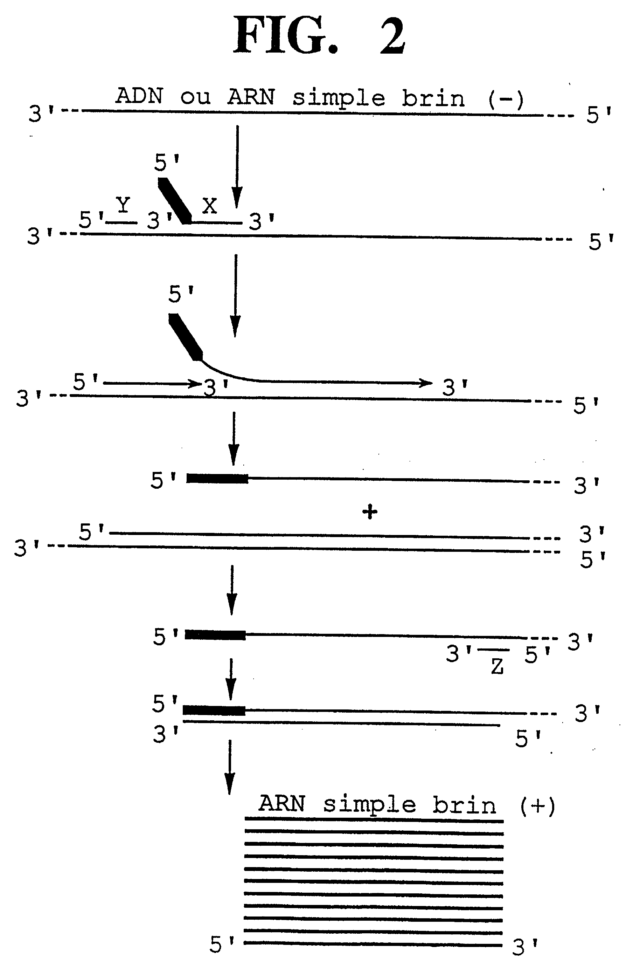

La figure 2 décrit une méthode d'installation d'un promoteur d'ARN polymérase par déplacement de brin pour l'obtention d'ARNs complémentaires d'une séquence cible (ARN ou ADN) par transcription, en phase homogène (une seule étape et une seule température). La séquence du promoteur est matérialisée par des rectangles noirs et les ARNs sont représentés en caractère gras. L'amorce X comprend de 5' vers 3' une séquence comprenant la séquence sens d'un promoteur d'une ARN polymérase et une séquence s'hybridant avec la séquence aval de la cible, l'amorce Z représente l'amorce dont l'élongation permet de générer le brin complémentaire du fragment obtenu à partir de l'amorce X, et l'amorce Y représente l'amorce de "déplacement" s'hybridant en aval de X et dont l'élongation produit le déplacement du produit d'élongation de X. Les signes "+" et "-" font référence à des séquences appartenant à des brins opposés d'un acide nucléique, c'est à dire des brins complémentaires.

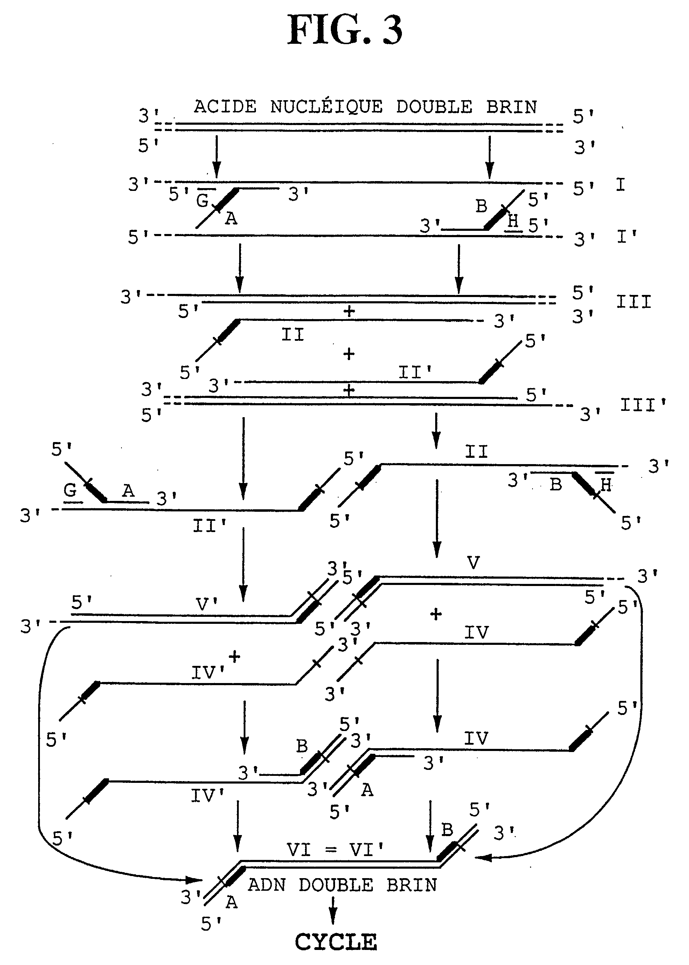

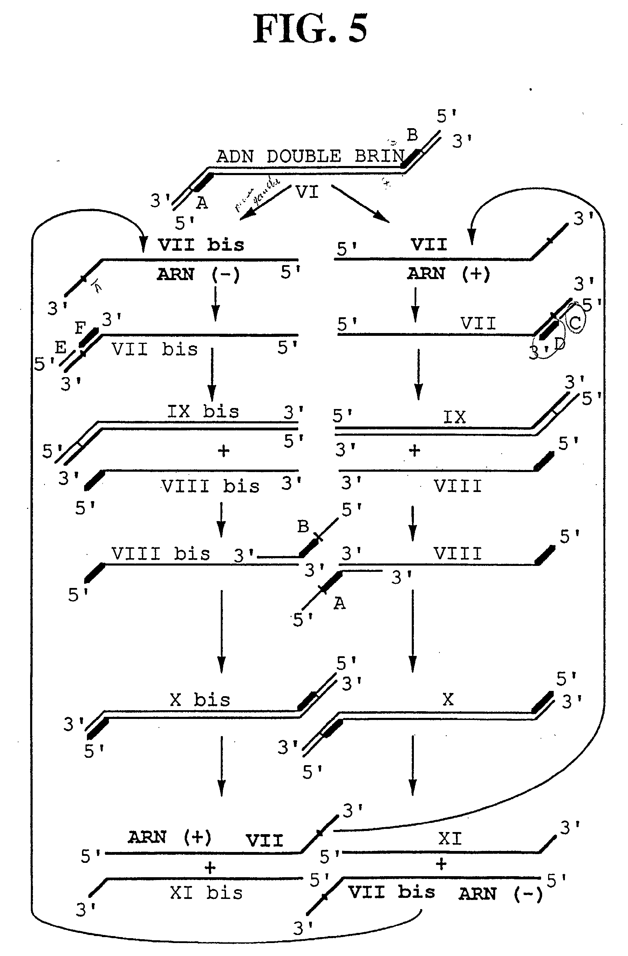

La figure 3 décrit une voie d'entrée de la méthode d'amplification de l'invention, par "Réaction de Transcription utilisant le Déplacement", à partir d'ADN ou d'ARN, simple ou double brin, homoduplex ou hétéroduplex décrite dans la figure 5 utilisant des amorces A et B comprenant une séquence sens promotrice complète représentée par un rectangle noir, qui, sous forme de double brin, produit un promoteur fonctionnel.

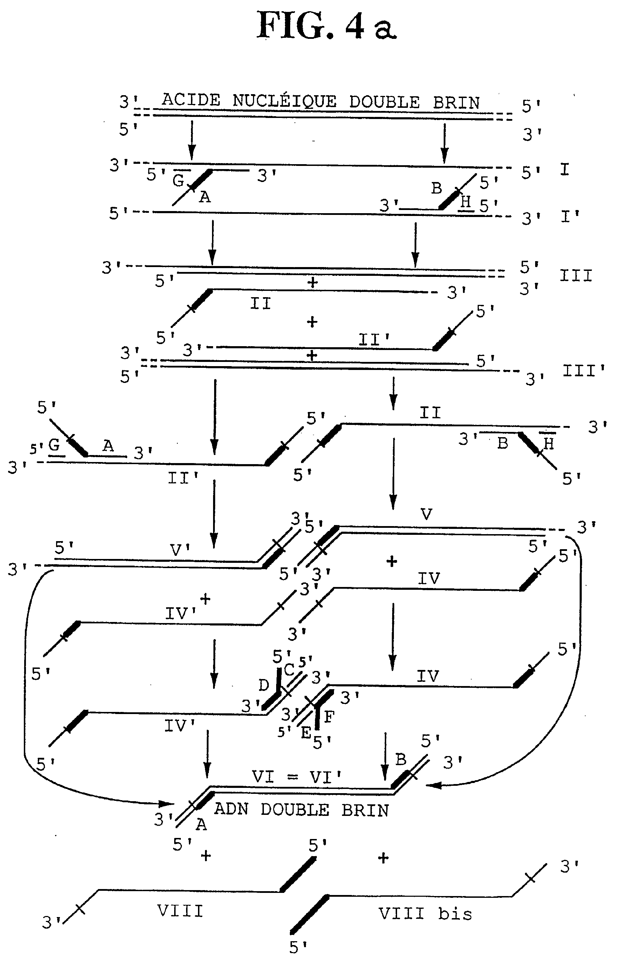

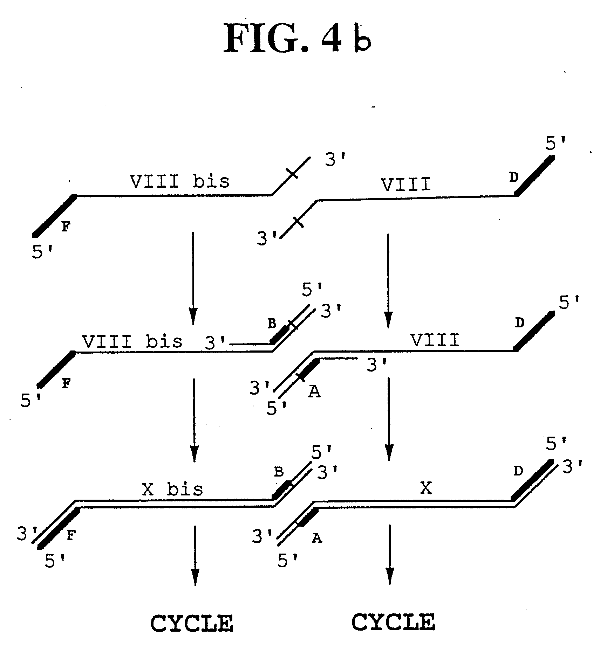

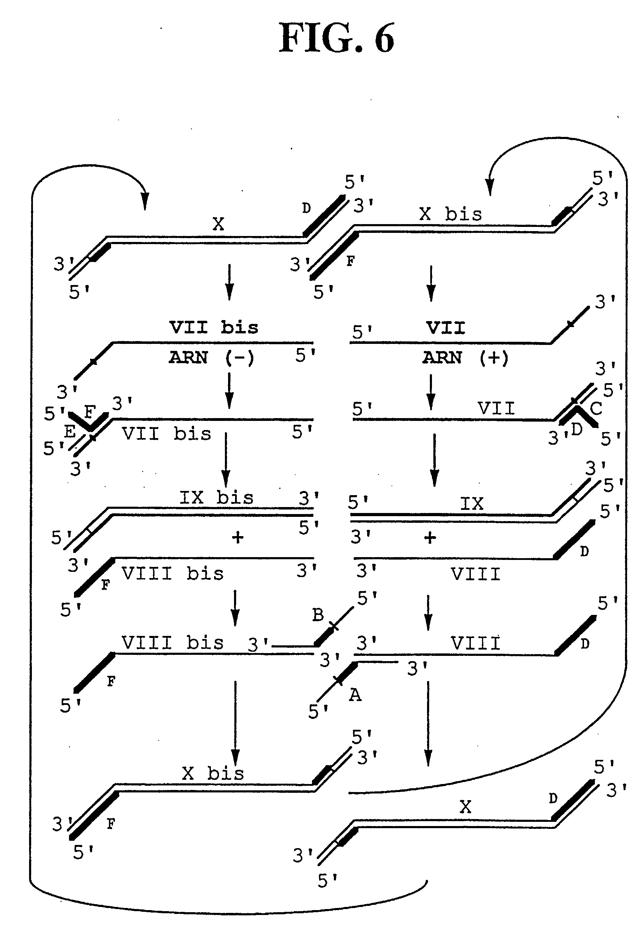

La figure 4 décrit une autre voie d'entrée de la méthode d'amplification de l'invention, par "Réaction de Transcription utilisant le Déplacement", à partir d'ADN ou d'ARN, simple ou double brin, homoduplex ou hétéroduplex, décrite dans la figure 6, utilisant des amorces A et B comprenant seulement une partie 3' de la séquence sens d'un promoteur représentée par un rectangle noir court, au contraire des amorces D et F qui comprennent une séquence sens promoteur complète représentée par un rectangle noir long, c'est à dire que les séquences contenues dans les amorces A et B, dans le cas des figures 4 et 6, lorsqu'elles sont sous forme de double brin, ne forment par de promoteur fonctionnel.
La figure 5 décrit le cycle d'amplification de la "Réaction de Transcription utilisant le Déplacement" conduisant à l'accumulation exponentielle d'ARN et d'ADN correspondant aux deux brins complémentaires de la cible initiale, utilisant des amorces A, B, D et F comprenant toutes une séquence sens promotrice complète représentée par un rectangle noir, qui, sous forme de double brin, produit un promoteur fonctionnel.
La figure 6 décrit le cycle d'amplification de la "Réaction de Transcription utilisant le Déplacement" conduisant à l'accumulation exponentielle d'ARN et d'ADN correspondant aux deux brins complémentaires de la cible initiale, utilisant des amorces A et B comprenant seulement une partie 3' de la séquence sens d'un promoteur représentée par un rectangle noir court, au contraire des amorces D et F qui comprennent une séquence sens promoteur complète représentée par un rectangle noir long, c'est à dire que les séquences contenues dans les amorces A et B, dans le cas des figures 4 et 6, lorsqu'elles sont sous forme de double brin, ne forment pas de promoteur fonctionnel. Par conséquent, ce cycle d'amplification ne fait intervenir que des polynucléotides double brin portant un seul promoteur fonctionnel, permettant la transcription seulement d'un des deux brins.

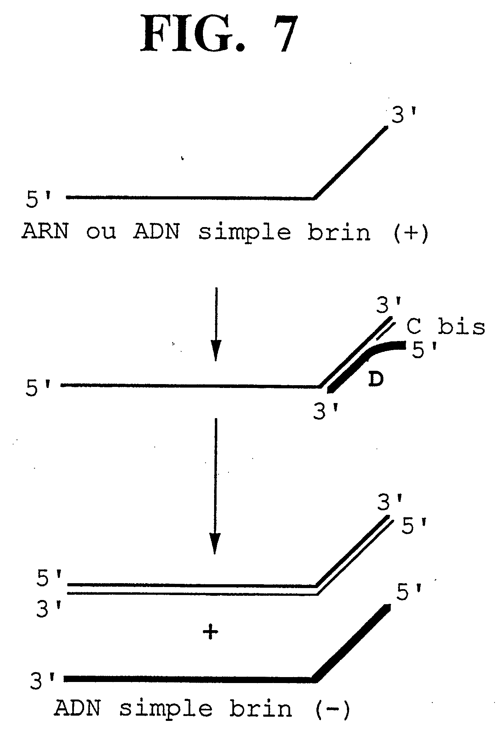

La figure 7 décrit un cas particulier d'amorces utilisables pour réaliser le cycle d'amplification par transcription utilisant le déplacement, comme décrit dans la figure 5. L'amorce D (caractère gras) correspond à celle décrite dans la figure 5 et l'amorce C bis est dans ce cas chevauchante avec l'amorce D. Ce cas de figure suppose que l'hybridation de l'amorce C bis sur la cible est favorisée sur le plan thermodynamique par rapport à l'hybridation de l'extrémité 5' de l'amorce D.

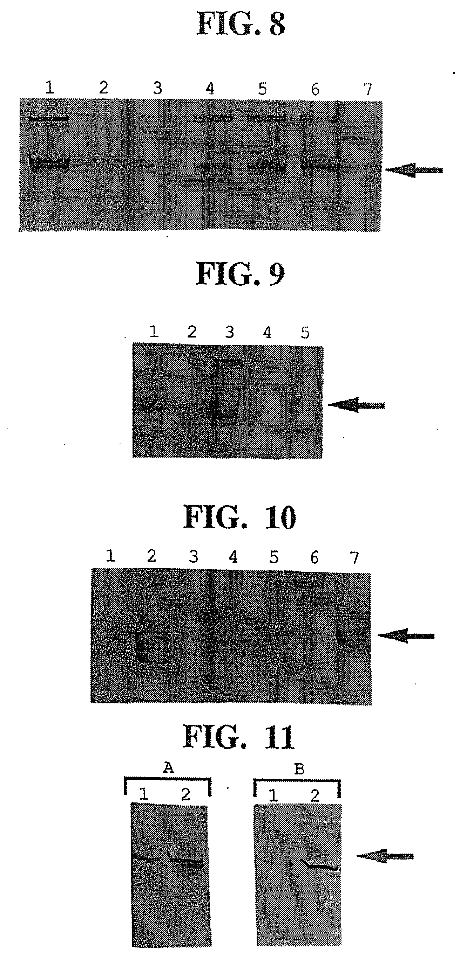

La figure 8 représente l'analyse des produits de transcription utilisant le déplacement, par séparation électrophorétique en gel de polyacrylamide, transfert sur membrane et hybridation, comme décrit dans l'exemple 1. La flèche indique le produit de transcription attendu, de 263 bases. La piste 1 correspond au témoin de transcription. La piste 2 correspond à l'essai en l'absence d'amorce de déplacement. Les pistes 3 à 7 correspondent aux essais contenant 5 mM, 500 nM, 50 nM, 5 nM ou 0,5 nM, respectivement.
La figure 9 représente l'analyse des produits de transcription utilisant le déplacement, par séparation électrophorétique en gel de polyacrylamide, transfert sur membrane et hybridation, comme décrit dans l'exemple 2. La flèche indique le produit de transcription attendu, de 263 bases. La piste 1 correspond au témoin de taille de 285 paires de bases obtenu par PCR à l'aide des amorces A24 (SEQ ID n° : 2) et 1028 (SEQ ID n° : 3). Les essais ont été réalisés en l'absence de transcriptase inverse et ARN polymérase T7 (piste 2) ou en présence des enzymes, avec les trois types d'amorces X, Y et Z (piste 3), sans l'amorce de déplacement Y (piste 4) ou sans amorce Z (piste 5).
La figure 10 représente l'analyse des produits de transcription utilisant le déplacement, par séparation électrophorétique en gel de polyacrylamide, transfert sur membrane et hybridation, comme décrit dans l'exemple 3. La piste 1 correspond au témoin de taille de 285 paires de bases obtenu par PCR à l'aide des amorces A24 (SEQ ID n° : 2) et 1028 (SEQ ID n° : 3). La piste 2 correspond au témoin de transcription de la cible de 285 paires de bases en présence d'ARN polymérase T7. Les pistes 3 à 6 correspondent aux essais réalisés en l'absence d'un des réactifs, à savoir l'ARN polymérase T7 (piste 3), la transcriptase inverse (piste 4), l'amorce Z (piste 5) ou l'amorce de déplacement Y (piste 6). La piste 7 correspond à l'essai en présence de tous les réactifs, comme décrit dans l'exemple 3.

La figure 11 représente l'analyse des produits de transcription à partir d'un acide nucléique encadré par deux promoteurs orientés pour la transcription de l'un ou de l'autre des brins de l'acide nucléique par séparation électrophorétique en gel de polyacrylamide, transfert sur membrane, et hybridation soit par une sonde A28 (SEQ ID n°: 5) (panel A), soit par une sonde A19 (SEQ ID n° : 11) (panel B), détectant respectivement soit les ARNs + soit les ARNs -, comme décrit dans l'exemple 4. La piste 1 correspond au témoin de taille de 275 paires de bases. La piste 2 correspond aux essais transcriptionnels.

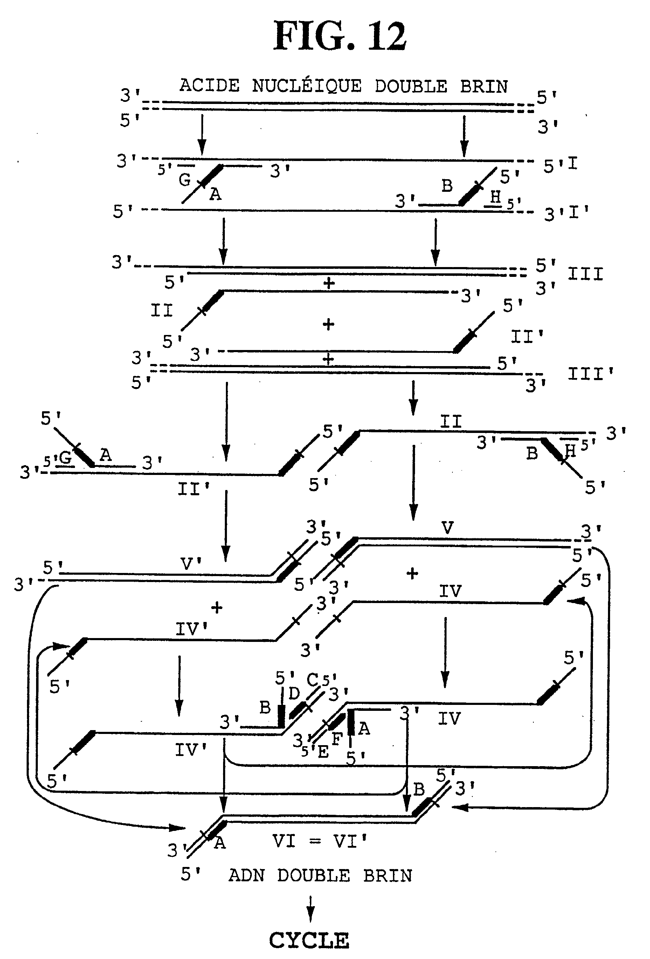

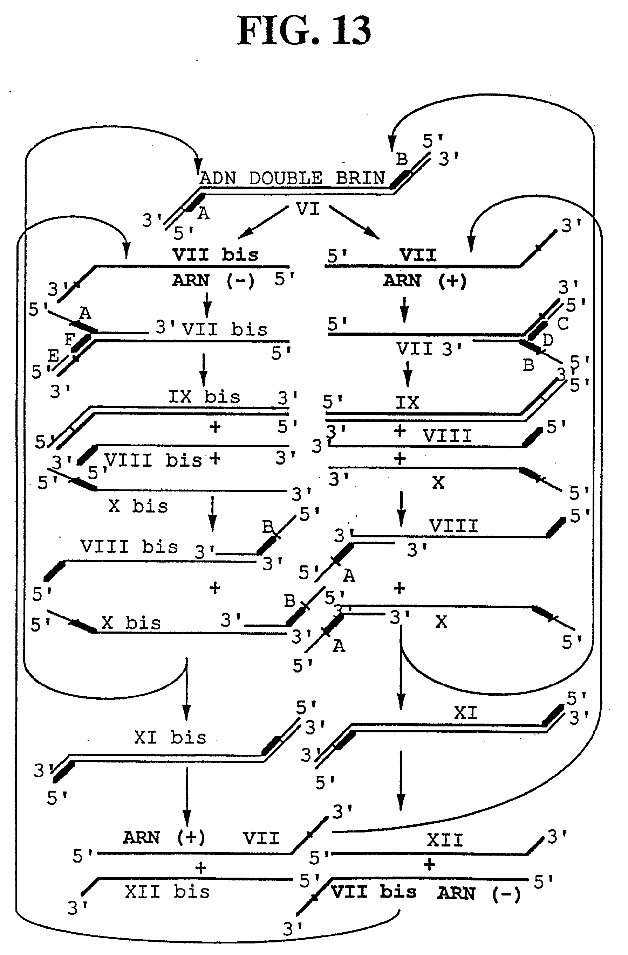

Les figures 12 et 13 représentent un autre mode de réalisation des figures 3 et 5, respectivement.

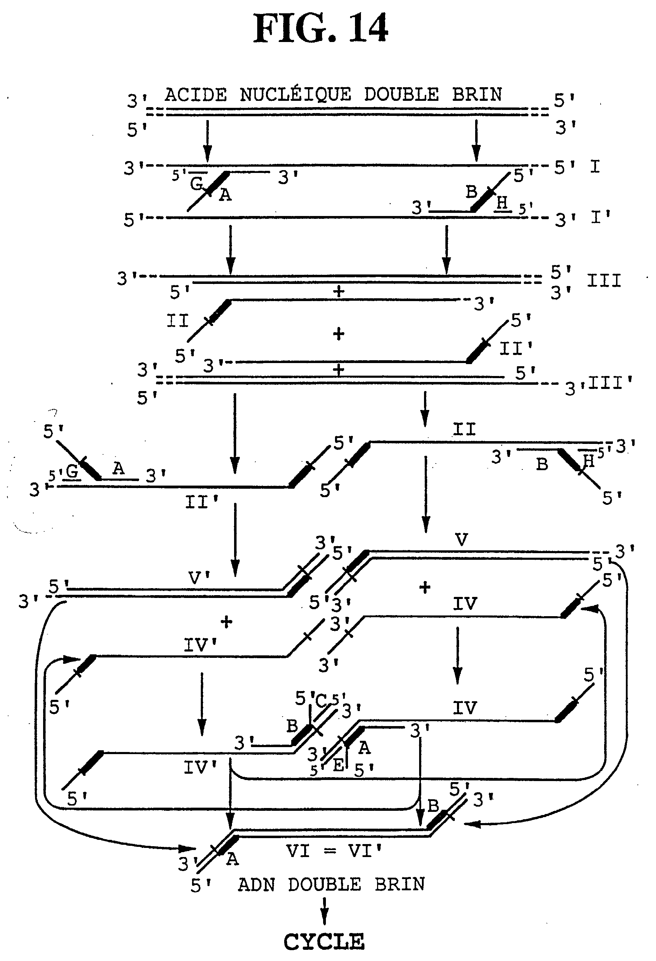

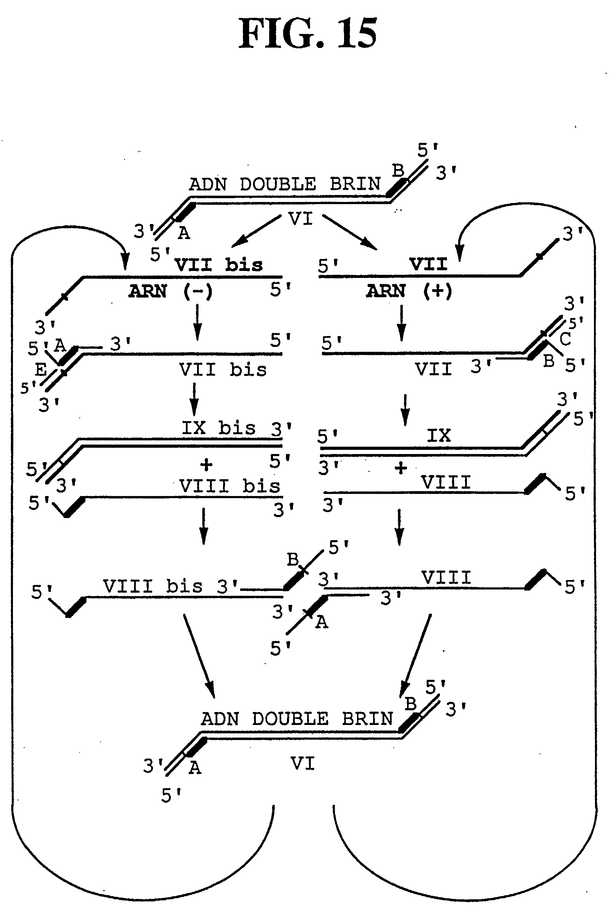

Les figures 14 et 15 représentent une variante simplifiée de la méthode d'amplification décrite dans les figures 3 et 5, respectivement ; à savoir, que l'amorce(s) promotrice(s) D et/ou F (facultative) est enlevée du milieu réactionnel.

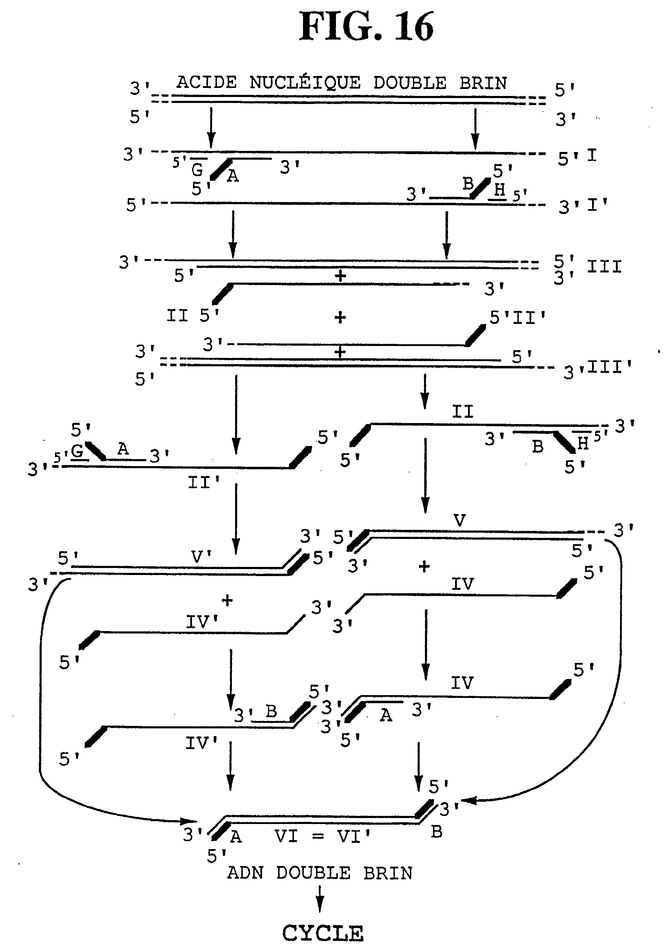

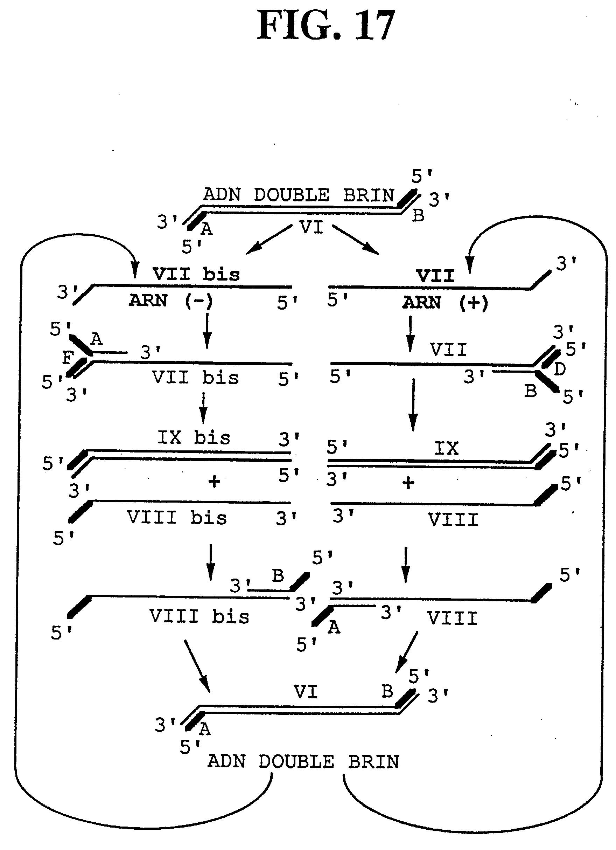

Les figures 16 et 17 représentent une variante simplifiée de la méthode d'amplification décrite dans les figures 3 et 5, respectivement ; à savoir, les amorces A et B ne contiennent plus la séquence définie facultative en amont de la séquence sens du promoteur. Par conséquent, l'utilisation des amorces de déplacement C et E (figure 5) n'est plus nécessaire. Le rôle de déplacement enzymatique est assuré par les séquences sens des promoteurs inclus dans A et B, soit les amorces D et F, respectivement.

Un procédé d'amplification d'une séquence d'un acide nucléique cible, ladite séquence comprenant, à partir de son extrémité 5', dans la direction 5'-3', une séquence amont ayant au moins 5 nucléotides et à partir de son extrémité 3' dans la direction 3'-5', une séquence aval ayant au moins 5 nucléotides, peut comprendre les étapes consistant :
- à obtenir un polynucléotide comprenant un premier segment correspondant à la séquence à amplifier et comprenant en outre au moins un deuxième segment choisi parmi des segments comprenant la séquence sens ou la séquence anti-sens d'un promoteur d'ARN polymérase ou au moins une partie de ladite séquence sens ou anti-sens, étant entendu qu'un tel deuxième segment comprenant ladite séquence sens ou une partie de celle-ci est situé en amont de l'extrémité 5' dudit premier segment, et qu'un tel deuxième segment comprenant ladite séquence anti-sens ou une partie de celle-ci est situé en aval de l'extrémité 3' dudit premier segment, et
- à mettre en contact ledit polynucléotide, en présence d'un système ayant une activité d'ARN polymérase, une activité d'ADN polymérase ARN-dépendante, une activité d'ADN polymérase ADN-dépendante et une activité de déplacement de brin, dans des conditions permettant le fonctionnement desdites activités, et en présence d'un excès de désoxyribonucléosides triphosphates et de ribonucléosides triphosphates, avec un ensemble d'amorces, présentes en excès, comprenant :
   a) une première amorce comprenant successivement depuis son extrémité 5' vers son extrémité 3' :
      - un premier segment polynucléotidique de séquence arbitraire, et dont la présence comme constituant de la première amorce est facultative, ledit premier segment, lorsqu'il est présent, comprenant au moins 5 nucléotides,
      - un second segment comprenant tout ou partie d'une séquence sens d'un promoteur d'une ARN polymérase, ou comprenant au moins une partie de ladite séquence sens incluant sa partie 3'-terminale,
      - et un troisième segment ayant la même longueur que ladite séquence amont et étant soit homologue de ladite séquence amont, soit capable de s'hybrider avec ladite séquence amont,
      et/ou
   b) une seconde amorce comprenant successivement depuis son extrémité 5' vers son extrémité 3' :
      - un premier segment, facultatif, défini comme le premier segment de la première amorce, mais non nécessairement identique à celui-ci,
      - un second segment défini comme le second segment de la première amorce, mais non nécessairement identique à celui-ci,
      - et un troisième segment ayant la même longueur que ladite séquence aval et étant soit homologue de ladite séquence aval, soit capable de s'hybrider avec ladite séquence aval, étant entendu que le troisième segment de l'une des première et seconde amorces est homologue de l'une des séquences amont ou aval de ladite séquence à amplifier, tandis que le troisième segment de l'autre amorce est capable de s'hybrider avec l'autre séquence aval ou amont,
      et/ou
   c) une troisième et/ou une quatrième amorces facultatives comprenant :
      soit une séquence homologue du second segment de la première amorce et de la seconde amorce, respectivement, et contenant au moins une partie d'une séquence sens dudit promoteur d'ARN polymérase incluant sa partie 5'-terminale,
      soit une séquence homologue d'une partie du premier segment de la première et seconde amorce respectivement, mais ne comprenant pas leur partie 5',
      et/ou
   d) une cinquième et/ou une sixième amorces facultatives 5 comprenant :
      soit une séquence homologue d'une partie de la troisième et de la quatrième amorces, respectivement, ladite partie ne comprenant pas le nucléotide 3'-terminal de ladite troisième ou quatrième amorce,
      soit une séquence homologue d'au moins une partie dudit premier segment de la première et de la deuxième amorces, respectivement.

Selon un mode de réalisation particulier du procédé d'amplification décrit précédemment, celui-ci comprend les étapes consistant :
- à obtenir un polynucléotide comprenant un premier segment correspondant à la séquence à amplifier et comprenant en outre au moins un deuxième segment choisi parmi des segments comprenant la séquence sens ou la séquence anti-sens d'un promoteur d'ARN polymérase ou au moins une partie de ladite séquence sens ou anti-sens, étant entendu qu'un tel deuxième segment comprenant ladite séquence sens ou une partie de celle-ci est situé en amont de l'extrémité 5' dudit premier segment, et qu'un tel deuxième segment comprenant ladite séquence anti-sens ou une partie de celle-ci est situé en aval de l'extrémité 3' dudit premier segment, et
- à mettre en contact ledit polynucléotide, en présence d'un système ayant une activité d'ARN polymérase, une activité d'ADN polymérase ARN-dépendante, une activité d'ADN polymérase ADN-dépendante, et une activité de déplacement de brin, dans des conditions permettant le fonctionnement desdites activités, et en présence d'un excès de désoxyribonucléosides triphosphates et de ribonucléosides triphosphates, avec un ensemble d'amorces, présentes en excès, comprenant :
   a) une première amorce comprenant successivement depuis son extrémité 5' vers son extrémité 3' :
      - un premier segment polynucléotidique de séquence arbitraire, et dont la présence comme constituant de la première amorce est facultative, ledit premier segment, lorsqu'il est présent, comprenant au moins 5 nucléotides,
      - un second segment comprenant tout ou partie d'une séquence sens d'un promoteur d'une ARN polymérase, ou comprenant au moins une partie de ladite séquence sens incluant sa partie 3'-terminale,
      - et un troisième segment ayant la même longueur que ladite séquence amont et étant soit homologue de ladite séquence amont, soit capable de s'hybrider avec ladite séquence amont,
   b) une seconde amorce comprenant successivement depuis son extrémité 5' vers son extrémité 3' :
      - un premier segment, facultatif, défini comme le premier segment de la première amorce, mais non nécessairement identique à celui-ci,
      - un second segment défini comme le second segment de la première amorce, mais non nécessairement identique à celui-ci,
      - et un troisième segment ayant la même longueur que ladite séquence aval et étant soit homologue de ladite séquence aval, soit capable de s'hybrider avec ladite séquence aval, étant entendu que le troisième segment de l'une des première et seconde amorces est homologue de l'une des séquences amont ou aval de ladite séquence à amplifier, tandis que le troisième segment de l'autre amorce est capable de s'hybrider avec l'autre séquence aval ou amont,
   c) une troisième et/ou une quatrième amorces comprenant :
      soit une séquence homologue du second segment de la première amorce et de la seconde amorce, respectivement, et contenant au moins une partie d'une séquence sens dudit promoteur d'ARN polymérase incluant sa partie 5'-terminale,
      soit une séquence homologue d'une partie du premier segment de la première et seconde amorce, respectivement, mais ne comprenant pas leur extrémité 5',
      et/ou
   d) une cinquième et/ou une sixième amorces comprenant :
      soit une séquence homologue d'une partie de la troisième et de la quatrième amorces, respectivement, ladite partie ne comprenant pas le nucléotide 3'-terminal de ladite troisième ou quatrième amorce,
      soit une séquence homologue d'au moins une partie dudit premier segment de la première et de la deuxième amorces, respectivement.

Dans la présente demande, l'expression "amont" désigne une région située du coté de l'extrémité 5' de l'acide nucléique ou de la séquence polynucléotidique dont il s'agit, et l'expression "aval" désigne une région située du côté de l'extrémité 3' dudit acide nucléique ou de ladite séquence polynucléotidique.

Par séquence homologue d'une autre séquence, on désigne une séquence identique à une autre, ou assez identique pour s'hybrider avec une séquence strictement complémentaire de la séquence avec laquelle elle est homologue.

Le terme "hétéroduplex" signifie un hybride ARN/ADN. Le terme "homoduplex" désigne un hybride ADN/ADN ou ARN/ARN.

Le terme "nucléosides triphosphates" désigne indifféremment des désoxyribonucléosides triphosphates et/ou des ribonucléosides triphosphates.

Dans le procédé qui vient d'être défini, le produit de départ comprend un premier segment "correspondant" à la séquence à amplifier, ce qui signifie qu'il s'agit soit de ladite séquence à amplifier, soit du brin complémentaire de ladite séquence à amplifier, étant entendu que le procédé fournit de toute façon une amplification des deux brins complémentaires, même lorsque le produit de départ est monobrin.

La capacité de déplacement de brin qui est bien connue pour certaines polymérases est associée, entre autres, à la synthèse d'ADN pour une ADN polymérase ADN-dépendante et la synthèse d'ADN par une ADN polymérase ARN-dépendante. Bien entendu, cette capacité de déplacement de brins est plus efficace lorsque les polymérases concernées n'ont pas d'activité de 5'-3' exonucléase. Cette capacité de déplacement de brin peut être apportée indépendamment des polymérases, comme défini ci-après.

Il convient de remarquer que les polymérases utilisées peuvent avoir une activité de ribonucléase H, dans la mesure où cette activité n'empêche pas le fonctionnement du procédé d'amplification.

Il est facile de voir que dans le procédé qui vient d'être décrit, les troisième et quatrième amorces peuvent être identiques. De même les cinquième et sixième amorces peuvent être identiques.

Pour que le procédé d'amplification décrit ci-dessus puisse fonctionner, il est nécessaire que la troisième et la quatrième amorces, si elles sont présentes, comprennent au moins la partie 5' de la séquence sens du promoteur d'ARN polymérase. C'est le cas de l'amorce D représentée aux figures 4, 5, 6 et 7 et de l'amorce F représentée aux figures 4, 5 et 6. Ces figures 4 et 6 montrent qu'il n'est pas nécessaire que l'amorce A et/ou B contienne une séquence complète du promoteur de l'ARN polymérase. Les produits de réaction issus de A et F d'une part, et B et D d'autre part doivent contenir une séquence sens fonctionnelle de promoteur d'ARN polymérase.

Par "activité de déplacement de brin", on désigne le phénomène par lequel un agent biologique, chimique ou physique, par exemple une ADN polymérase, provoque la dissociation d'un acide nucléique apparié de son brin complémentaire dans une direction de 5' vers 3', en conjonction avec, et à proximité de, la synthèse d'acide nucléique matrice-dépendante. Le déplacement de brin commence à l'extrémité 5' d'une séquence d'acide nucléique appariée et l'enzyme procède par conséquent à la synthèse d'acide nucléique immédiatement en 5' du site de déplacement. L'acide nucléique néo-synthétisé et l'acide nucléique déplacé ont généralement la même séquence nucléotidique qui est complémentaire du brin d'acide nucléique matrice. L'activité de déplacement de brin peut résider sur la même molécule que celle conférant l'activité de synthèse d'acide nucléique, et particulièrement la synthèse d'ADN, ou elle peut être une activité séparée et indépendante. Des ADN polymérases telles que l'ADN polymérase I d'*E. coli*, le fragment de Klenow de l'ADN polymérase I, l'ADN polymérase du bactériophage T7 ou T5, la transcriptase inverse du virus HIV sont des enzymes qui possèdent à la fois l'activité polymérase et l'activité de déplacement de brin. Des agents tels que les hélicases peuvent être utilisés en conjonction avec des agents inducteurs qui ne possèdent pas d'activité de déplacement de brin, afin de produire l'effet de déplacement de brin, c'est à dire le déplacement d'un acide nucléique couplé à la synthèse d'un acide nucléique de même séquence. De même, des protéines telles que Rec A ou la Single Strand Binding Protein d'*E. coli* ou d'un autre organisme pourraient être utilisées pour produire ou favoriser le déplacement de brin, en conjonction avec d'autres agents inducteurs. Pour plus de détails et une discussion du déplacement de brin, consulter KORNBERG, A. et BAKER T.A. (1992, DNA Replication, 2nd Edition, pp 113-225, Freeman, New York).

Par promoteur d'une ARN polymérase, on désigne une séquence ou structure qui permet d'initier la transcription.

A titre d'exemple, on peut citer des "Structure-boucle" capables d'initier la transcription (Mφllegaard, N. et al., (1994) PNAS, vol. 91, 3892-3895).

Par séquence sens d'un promoteur d'ARN polymérase, on désigne la séquence du promoteur double brin dont l'extrémité 3' est contiguë au site d'initiation de la transcription qui est défini par ce même promoteur.

Par séquence anti-sens d'un promoteur d'ARN polymérase, on désigne la séquence du promoteur double brin dont l'extrémité 5' est contiguë du nucléotide complémentaire du site d'initiation de la transcription qui est défini par ce même promoteur.

Selon un mode de réalisation particulier, le polynucléotide utilisé comme produit de départ comprend en outre :
a) en amont de l'extrémité 5' dudit deuxième segment comprenant tout ou partie de ladite séquence sens dudit polynucléotide, un segment homologue du premier segment de l'une des première et deuxième amorces,
   et/ou
b) en aval de l'extrémité 3' dudit deuxième segment, comprenant ladite séquence anti-sens, dudit polynucléotide, un segment capable de s'hybrider avec le premier segment de l'une des première et seconde amorces.

Il est aisé de vérifier que les divers produits de départ qui viennent d'être définis peuvent donner lieu à une réaction d'amplification.

Ces produits de départ peuvent être préparés le cas échéant par synthèse nucléotidique ou par hémisynthèse.

Dans un mode de réalisation particulier, le polynucléotide de départ peut être un ARN ou un ADN isolé d'un échantillon biologique. Dans ce cas, il est possible d'obtenir une réaction d'amplification telle que définie précédemment, au départ de l'acide nucléique cible, en une seule étape en ajoutant tous les réactifs (amorces, polymérases, etc.) dès le début de la réaction éventuellement après dénaturation de la cible. Ce mode de réalisation particulier est caractérisé par le fait que, pour obtenir ledit polynucléotide utilisé comme produit de départ dans le procédé défini précédemment, on opère au départ d'un acide nucléique cible contenant la séquence à amplifier et se prolongeant, au-delà de l'extrémité 3' de ladite séquence à amplifier, par une région aval, et au-delà de l'extrémité 5' de ladite séquence à amplifier, par une région amont,
on met en contact ledit acide nucléique en présence d'un excès de nucléosides triphosphates et en présence :
- dudit système à activités ADN polymérase, ARN polymérase et déplacement de brin,
- et d'un ensemble d'amorces contenant des amorces telles que définies précédemment et contenant en outre une septième amorce capable de s'hybrider avec ladite région aval de la cible, et une huitième amorce capable de s'hybrider avec une séquence complémentaire de ladite région amont.

Dans un mode de réalisation particulier, et afin de limiter le nombre d'amorces nucléotidiques utilisées, on peut choisir la séquence du premier segment de la première et/ou de la deuxième amorce comme équivalente à la séquence de la septième et/ou la huitième amorce.

Un ensemble d'amorces peut être utilisé pour la mise en oeuvre d'un procédé d'amplification d'un acide nucléique cible, tel que défini précédemment. Cet ensemble d'amorces peut comprendre au moins :
a) une amorce A1 comprenant successivement depuis son extrémité 5' vers son extrémité 3' :
   - un premier segment polynucléotidique facultatif, de séquence arbitraire, ledit premier segment, lorsqu'il est présent, comprenant au moins 5 nucléotides,
   - un second segment comprenant tout ou partie d'une séquence sens d'un promoteur d'une ARN polymérase, ou comprenant au moins une partie de ladite séquence sens incluant son extrémité 3'-terminale,
   - et un troisième segment capable de s'hybrider avec une séquence de la cible,
   et/ou
b) une amorce A2 comprenant :
   soit une séquence homologue de tout ou partie du second segment de l'amorce A1, et comprenant au moins la partie 5' de la séquence sens dudit promoteur d'ARN polymérase,
   soit une séquence homologue d'une partie du premier segment de la première et la seconde amorce mais ne comprenant pas leur partie 5', respectivement,
   et/ou
c) une amorce A3 comprenant :
   soit une séquence homologue d'une partie de l'amorce A2, ne comprenant pas le nucléotide 3'-terminal de A2,
   soit une séquence homologue d'au moins une partie d'un premier segment tel que défini ci-dessus présent sur A1.

Selon un mode de réalisation particulier, l'ensemble d'amorces comprend au moins :
a) une amorce A1 comprenant successivement depuis son extrémité 5' vers son extrémité 3' :
   - un premier segment polynucléotidique facultatif, de séquence arbitraire, ledit premier segment, lorsqu'il est présent, comprenant au moins 5 nucléotides,
   - un second segment comprenant tout ou partie d'une séquence sens d'un promoteur d'une ARN polymérase, ou comprenant au moins une partie de ladite séquence sens incluant son extrémité 3'-terminale,
   - et un troisième segment capable de s'hybrider avec une séquence de la cible,
b) une amorce A2 comprenant :
   soit une séquence homologue de tout ou partie du second segment de l'amorce A1, et comprenant au moins la partie 5' de la séquence sens dudit promoteur d'ARN polymérase,
   soit une séquence homologue d'une partie du premier segment de la première et la seconde amorce mais ne comprenant pas leur partie 5', respectivement,
   et/ou
   une amorce A3 comprenant :
   soit une séquence homologue d'une partie de l'amorce A2, ne comprenant pas le nucléotide 3'-terminal de A2,
   soit une séquence homologue d'au moins une partie d'un premier segment tel que défini ci-dessus, présent sur A1.

Le schéma de ce mode de réalisation particulier est donné à la figure 3 dans le cas d'un acide nucléique double brin ce qui nécessite bien entendu une dénaturation préalable.

Comme cela ressort de la figure 3, le produit d'élongation de l'amorce A le long de la cible (I) est déplacé par le produit d'élongation de l'amorce G, ce qui conduit à l'obtention du produit double brin (III) et de l'ADN monobrin (II). De même, le produit d'élongation de l'amorce B le long de l'autre brin (I') de la cible est déplacé par le produit d'élongation de H, ce qui conduit à l'obtention du produit double brin (III') et de l'ADN monobrin (II').

L'hybridation des amorces A et G sur l'ADN monobrin (II') conduit de façon analogue à l'obtention du produit ADN double brin (V') et de l'ADN monobrin (IV'). L'hybridation de l'amorce B sur l'ADN monobrin (IV') conduit au produit ADN double brin (VI) qui contient sur chacun de ses brins la séquence sens (ou la partie de séquence sens présente dans l'amorce A et dans l'amorce B, respectivement) d'un promoteur d'ARN polymérase.

On voit aisément que par des hybridations, élongations et déplacements similaires, le produit ADN monobrin (II) conduit au même produit ADN double brin (VI).

En outre, le produit double brin (V), s'il contient une séquence complète de promoteur d'ARN polymérase, donnera un produit de transcription ARN monobrin capable de s'hybrider avec B et H, et l'élongation par transcription inverse, avec déplacement, de ces amorces conduit à l'obtention d'un ADN monobrin dont il est facile de vérifier que par hybridation avec A suivie d'élongation, il permet d'obtenir également le produit ADN double brin (VI). Des réactions analogues, au départ de l'ADN double brin (V'), conduisent également au produit ADN double brin (VI). On voit sur la figure 3 que l'utilisation des septième et huitième amorces G et H conduit au produit ADN double brin (VI) qui est bien un polynucléotide utilisable comme produit de départ dans le procédé général qui a été décrit ci-dessus.

Dans un mode de réalisation particulier de procédé d'amplification d'acides nucléiques, en présence d'un fragment d'acide nucléique simple brin, une première amorce "A" (composée successivement, de 5' vers 3', d'une séquence arbitraire, suivie d'une séquence correspondant à tout ou partie d'une séquence ou d'un promoteur d'ARN polymérase, puis d'une séquence complémentaire de la cible) s'hybride sur sa séquence cible (I) (figure 3). En présence d'une ADN polymérase, des nucléotides sont ajoutés à l'extrémité 3' de cette amorce tout au long de la séquence cible (I). Le produit d'élongation est séparé du brin d'acide nucléique cible par déplacement de brin, suite à l'élongation d'une amorce "G" hybridée en amont de l'amorce A ce qui conduit à l'obtention du produit double brin (III) et de l'ADN monobrin (II). Une seconde amorce "B" (composée successivement, de 5' vers 3', d'une séquence arbitraire, suivie d'une séquence correspondant à tout ou partie d'une séquence d'un promoteur d'ARN polymérase, puis d'une séquence de l'acide nucléique cible) s'hybride à l'extrémité 3' du produit d'élongation néosynthétisé et libéré dans le milieu (II). En présence de l'ADN polymérase, des nucléotides sont ajoutés à l'extrémité 3' de cette amorce tout au long de la séquence du brin néosynthétisé. Ce second brin néosynthétisé est séparé du premier par déplacement de brin, suite à l'élongation d'une amorce "H" hybridée en amont de l'amorce B sur la cible. La première amorce A peut alors s'hybrider sur ce second brin libéré et être élongée en présence d'une polymérase et de désoxyribonucléosides triphosphates (dNTPs). Le produit de réaction double brin de taille définie qui en résulte (VI) contiendra alors à chaque extrémité une séquence promotrice fonctionnelle d'ARN polymérase, précédée en 5' par une séquence arbitraire (figure 3).

Le produit VI obtenu est le produit de départ de l'amplification décrite (figure 5).

Une ARN polymérase transcrit alors à partir du promoteur dont la séquence sens est incluse dans A, en présence de ribonucléosides triphosphates (rNTPs), des ARNs (VII) comportant donc à leur extrémité 3' la séquence complémentaire de l'amorce B (figure 5). Chaque ARN transcrit peut s'hybrider simultanément à son extrémité 3' une amorce "C" possédant à son extrémité 3' une séquence comportant tout ou partie de la séquence arbitraire de l'amorce B, et en aval de C, une amorce "D" possédant à son extrémité 3' tout ou partie de la séquence sens du promoteur qui est inclus dans B. L'ADN polymérase ARN-dépendante élonge, en présence de dNTPs, l'extrémité 3' de l'amorce C et déplace simultanément le brin en aval résultant de l'élongation de l'amorce D. On aboutit à la libération dans le milieu d'un ADN simple brin (VIII) complémentaire de l'ARN transcrit à partir du promoteur de A et comprenant à son extrémité 5' la séquence du promoteur de B. L'extrémité 3' de ce brin peut alors s'hybrider avec l'amorce A. L'ADN polymérase élonge l'extrémité 3' de l'amorce A le long du brin d'ADN et l'extrémité 3' du brin d'ADN le long de l'amorce A. Le produit de réaction double brin (X) de taille définie qui en résulte contient alors, à une extrémité, la séquence promotrice fonctionnelle d'ARN polymérase incluse dans B, et à l'autre extrémité, la séquence promotrice fonctionnelle d'ARN polymérase incluse dans A, précédée en 5' par la séquence arbitraire. L'ARN polymérase transcrit alors à partir du promoteur de B, en présence de rNTPs, des ARNs (VII bis) comportant donc à leur extrémité 3' la séquence complémentaire de l'amorce A.

De même, une ARN polymérase transcrit alors à partir du promoteur inclus dans B contenu dans un brin du fragment d'acide nucléique double brin (VI) de taille définie portant à chaque extrémité une séquence promotrice fonctionnelle d'ARN polymérase précédée en 5' par une séquence définie, en présence de rNTPs, des ARNs (VII bis) comportant donc à leur extrémité 3' la séquence complémentaire de l'amorce A (figure 5). L'extrémité 3' de chaque ARN transcrit s'hybride simultanément avec une amorce "E" possédant à son extrémité 3' une séquence comportant tout ou partie de la séquence "amont" de l'amorce A, et avec une amorce "F" possédant à son extrémité 3' tout ou partie de la séquence de l'amorce A à partir de la séquence promotrice, E et F étant choisis de telle façon que l'amorce F s'hybride en aval (c'est-à-dire vers 3') de E. L'ADN polymérase ARN-dépendante élonge, en présence de dNTPs, l'extrémité 3' de l'amorce E et déplace simultanément le brin en aval résultant de l'élongation de l'amorce F. On aboutit à la libération dans le milieu d'un ADN (VIII bis) simple brin complémentaire de l'ARN transcrit à partir du promoteur de B et comprenant à son extrémité 5' la séquence du promoteur de A. Ce brin peut alors hybrider à son extrémité 3' l'amorce B. L'ADN polymérase élonge l'extrémité 3' de l'amorce B le long du brin d'ADN (VIII bis) et l'extrémité 3' du brin d'ADN (VIII bis) le long de l'amorce B. Le produit de réaction double brin (X bis) de taille définie qui en résulte contient alors, à une extrémité, la séquence promotrice fonctionnelle d'ARN polymérase incluse dans A, et à l'autre extrémité, la séquence promotrice fonctionnelle d'ARN polymérase incluse dans B, précédée en 5' par la séquence définie arbitraire. L'ARN polymérase transcrit alors à partir du promoteur de A, en présence de rNTPs, des ARNs (VII) comportant donc à leur extrémité 3' la séquence complémentaire de l'amorce B.

Les ARNs (VII) comportant à leur extrémité 3' la séquence complémentaire de l'amorce B sont identiques à ceux transcrits de la matrice constituée du fragment d'acide nucléique double brin (VI) de taille définie contenant à chaque extrémité une séquence promotrice fonctionnelle d'ARN polymérase, précédée en 5' par une séquence définie arbitraire, à partir du promoteur de A. Ils fixent donc les amorces C et D par hybridation et le cycle de réaction précédemment décrit se poursuit.

De même, les ARNs (VII bis) comportant à leur extrémité 3' la séquence complémentaire de l'amorce A sont identiques à ceux transcrits de la matrice constituée du fragment d'acide nucléique double brin (VI) de taille définie contenant à chaque extrémité une séquence promotrice fonctionnelle d'ARN polymérase, précédée en 5' par une séquence définie arbitraire, à partir du promoteur de "B". Ils fixent donc les amorces E et F par hybridation et le cycle de réaction précédemment décrit se poursuit.

Il en résulte une amplification exponentielle de la cible comprise entre les deuxièmes segments des amorces A et B résultant en une accumulation d'ADN et/ou d'ARN double brin correspondant à la cible amplifiée.

La méthode décrite présente les avantages d'être isotherme, de pouvoir n'utiliser que deux enzymes, de produire à la fois de l'ARN et de l'ADN, d'être réalisable aussi bien à partir de cible ARN et ADN sans extrémité définie, de ne pas être limitée par la nature de la séquence cible et de ne pas nécessiter d'activité (ribo)nucléase pour la séparation des brins d'un duplex. En outre, elle n'implique pas l'incorporation de nucléotides modifiés dans les produits d'amplification par les polymérases utilisées.

Un nécessaire pour la mise en oeuvre du procédé tel que défini précédemment, peut comprendre un ensemble d'amorces A₁, A₂, A₃ tel que défini précédemment et au moins un second ensemble d'amorces comprenant :
a) une amorce B1 comprenant successivement depuis son extrémité 5' vers son extrémité 3' :
   - un premier segment polynucléotidique facultatif, de séquence arbitraire, ledit premier segment, lorsqu'il est présent, comprenant au moins 5 nucléotides,
   - un second segment comprenant tout ou partie d'une séquence sens d'un promoteur d'une ARN polymérase, ou comprenant au moins une partie de ladite séquence sens incluant son extrémité 3'-terminale,
   - et un troisième segment capable de s'hybrider avec une séquence nucléotidique complémentaire d'une séquence de la cible située, sur la cible, en amont de la séquence avec laquelle l'amorce A1 est capable d'hybridation,
   et/ou
b) une amorce B2 comprenant :
   soit une séquence homologue de tout ou partie du second segment de l'amorce B1, et comprenant au moins la partie 5' de la séquence sens dudit promoteur d'ARN polymérase,
   soit une séquence homologue d'une partie du premier segment de la première et seconde amorce, mais ne comprenant pas leur partie 5', respectivement,
   et/ou
c) une amorce B3 comprenant :
   soit une séquence homologue d'une partie de l'amorce B2, ne comprenant pas le nucléotide 3'-terminal de B2,
   soit une séquence homologue d'au moins une partie d'un premier segment tel que défini ci-dessus, présent sur B1, ledit nécessaire contenant en outre éventuellement une septième et une huitième amorces telles que définies précédemment.

Selon un mode de réalisation particulier de ce nécessaire, le second ensemble d'amorces comprend :
a) une amorce B1 comprenant successivement depuis son extrémité 5' vers son extrémité 3' :
   - un premier segment polynucléotidique facultatif, de séquence arbitraire, ledit premier segment, lorsqu'il est présent, comprenant au moins 5 nucléotides,
   - un second segment comprenant tout ou partie d'une séquence sens d'un promoteur d'une ARN polymérase, ou comprenant au moins une partie de ladite séquence sens incluant son extrémité 3'-terminale,
   - et un troisième segment capable de s'hybrider avec une séquence nucléotidique complémentaire d'une séquence de la cible située, sur la cible, en amont de la séquence avec laquelle l'amorce A1 est capable d'hybridation,
b) une amorce B2 comprenant
   soit une séquence homologue de tout ou partie du second segment de l'amorce B1, et comprenant au moins la partie 5' de la séquence sens dudit promoteur d'ARN polymérase,
   soit une séquence homologue d'une partie du premier segment de la première et seconde amorce, mais ne comprenant pas leur partie 5', respectivement,
   et/ou
   une amorce B3 comprenant :
   soit une séquence homologue d'une partie de l'amorce B2 ne comprenant pas le nucléotide 3'-terminal de B2,
   soit une séquence homologue d'au moins une partie d'un premier segment tel que défini ci-dessus, présent sur B1, ledit nécessaire contenant en outre éventuellement une septième et une huitième amorces telles que définies précédemment.

Dans la présente invention, l'échantillon à analyser peut être isolé à partir de n'importe quelle matière de départ suspectée de contenir la séquence d'acide nucléique cible. Pour les animaux, et plus particulièrement les mammifères, l'origine de ces matières peut être le sang, la moelle osseuse, la lymphe, les tissus durs (foie, rate, rein, poumon, ovaires, etc.), le crachat, le frottis, les fèces, l'urine. D'autres origines de matières de départ peuvent être des plantes, des prélèvements du sol, des aliments, ainsi que toute autre source suspectée de contenir des organismes biologiques.

L'isolement des acides nucléiques de ces matières de départ peut être réalisé de diverses façons. Ces procédés comprennent l'utilisation de détergents conduisant à des lysats, l'utilisation d'enzymes (lysozyme, protéinase K, par exemple) le traitement aux ultrasons, l'agitation mécanique en présence de billes, ou l'utilisation d'une presse de French. Dans certains cas, il peut être nécessaire de purifier les acides nucléiques extraits afin de se débarrasser d'éventuels contaminants tels que des nucléases. Dans ce cas, la purification des acides nucléiques peut être réalisée par extraction au phénol-chloroforme, chromatographie, échange d'ions, électrophorèse, centrifugation à l'équilibre ou par capture par hybridation sur un support solide.

Dès que les acides nucléiques sont isolés, une fragmentation sommaire peut être réalisée par des moyens tels que le traitement aux ultrasons, afin d'obtenir des fragments de taille inférieure à 10 kilobases. Ceci permet alors de faciliter la dénaturation initiale, en particulier dans le cas d'un acide nucléique double brin.

Les amorces utilisées dans la méthode ont une longueur comprise entre 5 et 100 nucléotides. Par exemple, la longueur des amorces A et B est généralement comprise entre 30 et 100 nucléotides et préférentiellement comprise entre 40 et 70 nucléotides, tandis que la longueur des amorces C, D, E, F, G et H est comprise entre 5 et 50 nucléotides et préférentiellement de 10 à 35 nucléotides. Les amorces A et B doivent être substantiellement homologues ou complémentaires, respectivement, dans leur extrémité 3', d'une séquence de la cible (supposée ici monobrin), de telle façon que dans des conditions drastiques ou fortement discriminantes, leur hybridation sur leur site spécifique de la cible ou de son complémentaire puisse s'effectuer. Les amorces A et B peuvent contenir, à leur extrémité 5' une séquence définie arbitraire, d'une longueur d'au moins 5 nucléotides. Cette séquence peut éventuellement être commune à A et B mais peut tout aussi bien être différente. Ladite séquence définie peut être choisie indifféremment, pourvu qu'elle ne comporte pas d'homologie significative avec la séquence cible, et qu'elle ne donne pas lieu à la formation de dimères entre A et B, ni de structure secondaire (par exemple tige-boucle ou duplex) au sein de l'amorce. En aval de cette séquence définie (c'est à dire vers 3'), les amorces A et B doivent contenir une séquence sens d'une séquence qui avec son complémentaire a la capacité de fixer une ARN polymérase puis d'initier la transcription d'ARN. Ces séquences dites "promotrices" ou encore appelées sens ou anti-sens de "promoteurs" sont par exemple, des séquences promotrices d'ARN polymérases phagiques (par exemple des bactériophage T7, T3 et SP6). Il est connu que l'ARN polymérase du phage T7 requiert la présence d'un promoteur spécifique sur l'ADN pour une transcription efficace d'ARN. La séquence de ce promoteur spécifique est parfaitement caractérisée (Dunn et Studier, 1983. *J. Mol. Biol.* 166 : 477-535) et la grande spécificité de transcription de l'ARN polymérase T7 à partir de son promoteur a été mise en évidence (Bailey *et al.*, 1983. *Proc. Natl. Acad Sci.* 80 : 2814-2818). Ces propriétés peuvent être utilisées afin de produire *in vitro* des ARNs à partir de matrices contenant un promoteur double brin fonctionnel (Krupp et Söll, 1987. *FEBS Lett.* 2 : 271-275). De même, des ARNs peuvent être transcrits *in vitro* à l'aide d'ARN polymérase T7 à partir de matrices oligonucléotidiques synthétiques comportant un promoteur double brin (Milligan *et al.*, 1987. *Nucl. Acids Res*. 15 : 8783-8798). L'article de Krupp (1988 *Gene* 72 : 75-89) constitue une bonne revue des modes d'utilisation des ARN polymérases phagiques et des stratégies utiles pour la synthèse d'ARN *in vitro*. Les séquences promotrices utilisées peuvent aussi être celles de promoteurs d'ARN polymérases eucaryotes telles que l'ARN polymérase III (Sharp *et al.*, 1985. Crit. Rev. Biochem. 19 : 107-144).

Certaines des amorces du procédé défini précédemment comprennent des séquences homologues. Un ajustement des concentrations relatives des différentes amorces permet de favoriser l'hybridation différentielle pour le bon fonctionnement des cycles.

Dans la présente méthode, dès que des fragments d'acide nucléique cible sont obtenus, ceux-ci sont dénaturés, le cas échéant, afin de les rendre simple brin et de permettre l'hybridation des amorces A et G, et, si l'acide nucléique initial est double brin, B et H (ou *vice versa).* L'augmentation de la température à environ 95°C est préférable pour la dénaturation, mais la séparation des brins peut également être réalisée par augmentation du pH, puis neutralisation du milieu afin de permettre l'hybridation des amorces sur la cible. Avant ou après que les acides nucléiques cibles soient dénaturés, un mélange réactionnel contenant un excès de désoxyribonucléosides triphosphates et de ribonucléosides triphosphates, une ARN polymérase et une ADN polymérase sont ajoutées. Dans le cas ou l'élévation de température est utilisée pour dénaturer les acides nucléiques cibles, à moins d'utiliser des enzymes thermostables, il est préférable d'ajouter les enzymes après dénaturation. Le mélange réactionnel nécessaire à la réalisation de la réaction d'amplification selon l'invention peut également contenir des polyols tels que, par exemple, le glycérol, le sorbitol et le polyéthylèneglycol ou des agents dénaturants et/ou des solvants tels que le (diméthyl)formamide le diméthylsulfoxyde (DMSO). Ces réactifs peuvent en effet permettre de réduire les réactions d'hybridation non spécifiques qui pourraient engendrer un éventuel bruit de fond.

Les polymérases utilisées dans le procédé de l'invention doivent être préférentiellement pourvues d'une activité de déplacement de brin. Cette activité est une propriété bien connue de certaines ADN polymérases (Sambrook *et al.*, 1989. Molecular Cloning : A Laboratory Manual, 2nd Edition, pp. 5.33-5.35, Cold Spring Harbor Laboratory, Cold Spring Harbor). Les propriétés des ADN polymérases, et notamment de l'activité de déplacement de brin de certaines d'entre elles sont détaillées par Kornberg et Baker (1992. DNA Replication, 2nd Edition, pp. 113-225, Freeman, New York). L'activité de déplacement de brin a été mise en évidence initialement pour le fragment de Klenow de l'ADN polymérase I d'*Escherichia coli* (Masamune et Richardson, 1971. *J. Biol. Chem.* 246 : 2692-2701), ce qui confère à cette enzyme la capacité d'initier la réplication d'un acide nucléique à partir de l'extrémité 3'OH d'une césure sur un ADN double brin. Cette propriété de déplacement de brin est limitée dans le cas où les ADN polymérases possèdent une activité 5'-3' exonucléase (Lundquist et Olivera, 1982. *Cell* 31 : 53-60). Cette activité de déplacement de brin a également été mise en évidence chez des ADN polymérases thermostables telles que la *Tli* ADN polymérase (Kong *et al.*, 1993. *J. Biol. Chem.* 268 : 1965-1975). Dans ce cas, il a également été montré que des formes mutées de cette enzyme ne possédant pas d'activité 5'-3' exonucléase possèdent une plus grande capacité de déplacement de brin. Le déplacement de brin n'est pas une propriété commune à toutes les ADN polymérases, puisque certaines d'entre elles, comme la T4 ADN polymérases, ne sont pas capables de réaliser, seules, le déplacement de brin. Cette activité de déplacement de brin a également été mise en évidence pour la T7 ADN polymérase (Lechner *et al.*, 1983. *J. Biol. Chem.* 258 : 11174-11184) et pour la transcriptase inverse d'HIV (Huber *et al.*, 1989. *J. Biol. Chem.* 264 : 4669-4678). Les ADN polymérases ayant une capacité de déplacement de brin, et plus particulièrement capables d'initier la polymérisation (de 5' vers 3') à partir de l'extrémité 3'OH d'une césure d'un ADN double brin (figure 1) sont utiles pour la réalisation de la réaction d'amplification de la présente invention. De manière préférentielle, une ADN polymérase dépourvue d'activité 5'-3' exonucléase est utilisée pour la réalisation du cycle d'amplification, puisque l'efficacité de l'activité de déplacement de brin est supérieure chez des enzymes dépourvues de celle-ci. Le fragment de Klenow de l'ADN polymérase I d'*Escherichia coli* constitue un exemple de polymérase dépourvue d'activité 5'-3' exonucléase, de même que des polymérases telles que la T4 ADN polymérase, la T7 ADN polymérase ou la Sequenase (US Biochemical), la T5 ADN polymérase ou la Phi29 ADN polymérase pourraient également être utilisées. Néanmoins, la présente invention comprend également l'utilisation d'ADN polymérases possédant cette activité 5'-3' exonucléase, lorsque celle-ci n'empêche pas la réalisation de la méthode d'amplification. Dans ce cas, le rendement de la réaction d'amplification peut être amélioré par inhibition spécifique de l'activité 5'-3' exonucléase des ADN polymérases dans les conditions de réaction utilisées.

La présente méthode d'amplification peut nécessiter une étape de transcription inverse, afin de recopier un ARN transcrit en ADNc. Cette étape peut notamment se réaliser par l'utilisation d'une transcriptase inverse de type AMV (Avian Myeloblastosis Virus) ou MMLV (Moloney Murine Leukemia Virus) couramment disponibles commercialement. Tout autre enzyme possédant une activité ADN polymérase ARN- et/ou ADN-dépendante peut être utilisée dans la présente invention, pourvu qu'elle possède une activité de déplacement de brin. Dans le cas contraire, l'activité de déplacement de brin pourrait être conférée par un agent inducteur, une activité de type hélicase ou Rec A. Les propriétés de Rec A, notamment dans le processus de réassociation d'ADN simple brin, de capture de brin ou d'assimilation de brin sont détaillées par Mc ENTEE et WEINSTOCK dans The Enzymes, vol. XIV, pp. 445-470. L'étape de transcription inverse peut par exemple être réalisée à l'aide de l'ADN polymérase I d'*Escherichia coli*, car il a été démontré que cette enzyme possède également une activité ADN polymérase ARN-dépendante (Ricchetti et Buc, 1993. *EMBO* 12 : 387-396). La présente invention peut également utiliser dans ce but des ADN polymérases thermostables ARN- et/ou ADN-dépendantes telles que la Taq polymérase ou la *Tth* polymérase (pour une revue sur les propriétés des ADN polymérases thermostables, voir Rolf *et al.*, 1992. PCR : Clinical Diagnostics and Research, pp. 224-258, Springer-Verlag, Heidelberg).

Du fait de l'utilisation des propriétés de déplacement de brins des ADN polymérases, ou d'un autre agent inducteur associé, la méthode d'amplification d'acides nucléiques par transcription et déplacement, appelée par la suite méthode "DTR", ne nécessite pas d'activité nucléase, qu'il s'agisse d'activité endonucléase, exonucléase ou ribonucléase. En particulier, la méthode "DTR" ne nécessite pas l'utilisation d'activité RNase H, commune à diverses autres techniques d'amplification citées précédemment, activité conférée par certaines transcriptases inverses et devant être complétée par addition de RNase H d'*Escherichia coli*. Dans la méthode "DTR", on peut utiliser la transcriptase inverse MMLV, qui possède une activité RNase H plus faible que celle de l'AMV (Sambrook *et al.*, 1989. Molecular Cloning : A Laboratory Manual, 2nd Edition, pp. 5.52-5.55, 8.11, 8.17, Cold Spring Harbor Laboratory, Cold Spring Harbor), comme ADN polymérase ARN- et ADN-dépendante. Une forme de transcriptase inverse MMLV dépourvue d'activité RNase H peut également être utilisée dans la méthode "DTR". L'activité RNase H de la transcriptase inverse MMLV peut en effet être supprimée par délétion d'une partie du gène de structure de cette enzyme (Kotewicz *et al.*, 1988. *Nucl. Acids Res*. 16 : 265-277), conduisant à une polymérase d'efficacité accrue par rapport à la transcriptase inverse MMLV sauvage, et disponible commercialement sous le nom de "Superscript" (Gerard *et al.*, 1989. *Focus* 11 : 66-69). L'activité RNase H de la transcriptase inverse peut également être supprimée par réalisation de mutations ponctuelles de la partie du gène conférant cette activité à la polymérase (Gerard *et al.*, 1992. Focus 14 : 91-93), conduisant à une enzyme ayant également une efficacité accrue et un taux de polymérisation de l'ADN supérieur à la Superscript. Cette enzyme est également disponible commercialement sous le nom de "SuperscriptII" (GIBCO-BRL). Il en existe une autre forme disponible commercialement sous le nom de "StrataScript" (STRATAGENE).

Contrairement à diverses méthodes d'amplification, la présente invention ne nécessite pas de cycles de température afin de dissocier le brin néosynthétisé de sa matrice. Dans cette méthode, une seule température peut être employée, dès que la dénaturation initiale de la cible a eu lieu, le cas échéant. Cette température doit être suffisamment haute afin de définir des conditions discriminantes d'hybridation qui autorisent une hybridation spécifique des amorces sur leur cible. Cette température peut être située par exemple entre 37°C et 45°C avec des réactifs enzymatiques conventionnels, mais peut être plus élevée si l'on a recours à des enzymes thermostables.

Les figures 3 et 5 décrivent la méthode d'amplification par Réaction de Transcription utilisant le Déplacement. La figure 3 décrit une voie d'entrée possible de la méthode d'amplification et la figure 5 le cycle d'amplification proprement dit.

Après dénaturation initiale de l'acide nucléique cible (ADN ou ARN, simple brin ou double brin, homo- ou hétéroduplex), les amorces A, B, G et H sont hybridées sur leur brin d'acide nucléique respectif (figure 3). L'élongation simultanée de ces amorces en présence d'un excès de désoxyribonucléosides triphosphates et d'une ADN polymérase (ARN et/ou ADN dépendante, selon que la cible est de l'ADN ou de l'ARN) conduit au déplacement du brin d'ADN provenant de l'élongation de A par élongation du brin issu de G et le déplacement du brin provenant de l'élongation de B par élongation du brin issu de H. Les ADN simple brin obtenus par élongation des amorces A d'une part et B d'autre part peuvent alors hybrider les amorces B et H, et A et G, respectivement. L'élongation de G provoque le déplacement du brin s'élongeant à partir de l'amorce A, et l'élongation de H provoque le déplacement du brin s'élongeant à partir de l'amorce B. Les deux brins de longueur définie ainsi libérés sont parfaitement complémentaires et peuvent s'hybrider l'un sur l'autre, ou s'hybrider avec les amorces B (brin correspondant à l'élongation de A) ou A (brin correspondant à l'élongation de B), l'hybridation de ces amorces courtes étant plus favorable sur le plan thermodynamique. L'élongation de A et B sur leurs brins respectifs conduit alors à un fragment d'acide nucléique double brin de longueur définie correspondant à la séquence cible comprise entre les deuxièmes fragments des amorces A et B, et encadrée à chaque extrémité par une séquence définie suivie en aval d'un promoteur d'ARN polymérase.

La voie d'entrée précédemment présentée (figure 3) peut également être réalisée à partir d'une cible capturée par le biais d'une sonde fixée sur un support solide. Cette sonde peut être immobilisée de manière covalente ou passive, telle que décrite dans le brevet Français n° 91 09057 et le brevet International WO 91/19812. Cette immobilisation de sondes peut également être effectuée par le biais de (co)polymères, notamment un copolymère de N-vinylpyrrolidone auxquels sont couplées des sondes par formation d'un conjugué, lequel conjugué est immobilisé sur un support solide par adsorption passive. Plus particulièrement, les amorces A et/ou B, ou G et/ou H peuvent être fixées sur support solide, à condition que cette fixation ne soit pas réalisée par l'extrémité 3' des amorces, afin que l'extrémité 3' de ces amorces soit élongeable par une ADN polymérase en présence de désoxyribonucléosides triphosphates. Plus particulièrement, il peut être avantageux que tout ou partie des amorces A et G d'une part et B et H d'autre part soient reliées l'une à l'autre par le biais d'un bras de liaison quelconque (hydrocarbonée ou nucléotidique par exemple) et ce, à partir de leur extrémité 5'. Ceci peut permettre de contrôler et équilibrer le ratio de A vis à vis de G, d'une part, et de B vis à vis de H, d'autre part, dans la réaction d'amplification décrite dans la présente invention.

La matrice double brin ainsi obtenue permet de transcrire des ARN à partir de chaque promoteur encadrant la séquence cible (figure 5), en présence d'un excès de ribonucléosides triphosphates et de l'ARN polymérase correspondant à chaque promoteur. Pour simplifier la méthode « DTR », cette séquence promotrice peut être une séquence d'un promoteur d'une ARN polymérase phagique, ladite séquence promotrice étant la même sur les amorces A et B (par exemple, le consensus du promoteur de l'ARN polymérase du phage T7). Dans le cas où les amorces A et B contiennent la même séquence promotrice, une seule ARN polymérase peut être utilisée dans la réaction d'amplification.

L'ARN transcrit à partir du promoteur contenu dans A peut fixer les amorces nucléotidiques C et D. Ces amorces sont suffisamment homologues à une partie de B afin de pouvoir s'hybrider, dans les conditions de réaction, à l'ARN transcrit du promoteur de A. Ces amorces C et D peuvent être adjacentes, c'est à dire séparées sur la cible par un espace d'au moins un nucléotide ou être contiguës, c'est à dire que leurs extrémités 3' et 5' sont juxtaposées lorsqu'elles sont hybridées. Les amorces C et D peuvent également être chevauchantes, c'est à dire que l'extrémité 3' de l'amorce C possède une analogie de séquence avec l'extrémité 5' de l'amorce D. Dans le cas d'amorces chevauchantes, l'extrémité 3' de l'amorce C peut être suffisamment homologue à l'extrémité 5' de l'amorce D, pourvu que l'hybridation compétitive entre C et D aille au bénéfice de l'hybridation de l'extrémité 3' de C. La figure 7 décrit un cas particulier de l'invention, où les amorces C et D sont chevauchantes. Dans ce cas, l'amorce C (appelée C bis) possède la même séquence nucléotidique que l'extrémité 5' de l'amorce D. Afin de favoriser l'hybridation compétitive au profit de l'amorce C, surtout à son extrémité 3', celle-ci peut contenir des analogues de bases ou des nucléotides modifiés dont l'hybridation sur l'ARN conduit à un duplex de stabilité supérieure à celui engendré par l'hybridation de l'amorce D. Ces nucléotides modifiés comprennent également ceux modifiés au niveau de la liaison internucléotidique (comme par exemple les liaisons phosphorothioate, H-phosphonate, alkyl phosphonate), au niveau du squelette comme par exemple les alpha-oligonucléotides (brevet Français n° 2 607 507) ou les PNA (Egholm *et al.*, 1992. *J. Am. Chem. Soc.* 114 : 1895-1897). D'une manière générale, Il est cependant nécessaire d'établir des conditions dans lesquelles l'hybridation de C n'empêche pas l'hybridation de l'extrémité 3' de D. Dans le cas où C et D sont chevauchants, il est avantageux d'éviter que l'extrémité 3' de C soit hybridée trop près de celle de D. Un espacement de 5 nucléotides constitue une marge de sécurité préférée pour éviter que C n'empêche l'hybridation de D. Dans le cas d'un procédé d'amplification utilisant la transcription, il est préférable que l'amorce D contienne l'extrémité 5' de la séquence sens du promoteur inclus, même partiellement, dans l'amorce B, l'élongation ultérieure de D devant permettre la reconstitution d'un promoteur pouvant fixer une ARN polymérase et diriger l'initiation de la transcription. La longueur des amorces C et D peut être comprise entre 5 et 100 nucléotides et comprendre une partie de la séquence cible, mais ne dépassant pas la longueur de la région cible comprise entre les amorces A et B.

L'élongation simultanée des amorces C et D sur la matrice ARN, en présence d'une ADN polymérase comprenant notamment une activité ARN dépendante et d'un excès de désoxyribonucléosides triphosphates, conduit au déplacement du brin issu de D. Le brin d'ADN ainsi libéré peut s'hybrider avec l'amorce A. Une ADN polymérase (comprenant notamment une activité ADN- et/ou ARN-dépendante) peut alors élonger l'extrémité 3' de A sur le brin d'ADN et l'extrémité 3' du brin d'ADN sur l'amorce A. L'ADN double brin ainsi obtenu, de taille définie, possède alors le promoteur fonctionnel de l'amorce B, ainsi que le promoteur fonctionnel de l'amorce A, ledit promoteur étant de ce côté précédé d'une séquence arbitraire.

La matrice double brin ainsi obtenue permet de transcrire des ARNs à partir de chaque promoteur situé à l'extrémité de ce fragment de taille définie, en présence d'un excès de ribonucléosides triphosphates et de l'ARN polymérase correspondant à chaque promoteur. Les ARNs transcrits à partir du promoteur de B peuvent alors s'hybrider avec les amorces E et F.

Les amorces nucléotidiques E et F sont suffisamment homologues à une partie de A afin de pouvoir s'hybrider, dans les conditions de réaction, à l'ARN transcrit du promoteur de B. Comme C et D, les amorces E et F peuvent être adjacentes ou contiguës, ou chevauchantes. Dans le cas d'amorces chevauchantes, l'extrémité 3' de l'amorce E peut être suffisamment homologue à l'extrémité 5' de l'amorce F, pourvu que l'hybridation compétitive entre E et F soit au bénéfice de l'hybridation de l'extrémité de E. Afin de faciliter l'hybridation compétitive au profit de l'extrémité de l'amorce E, celle-ci peut contenir, comme C, des analogues de bases ou des nucléotides modifiés dont l'hybridation sur l'ARN conduit à un duplex de stabilité supérieure à celui engendré par l'hybridation de l'amorce F. Il est cependant nécessaire d'établir des conditions dans lesquelles l'hybridation de E n'empêche pas l'hybridation de l'extrémité 3' de F. Dans le cas où E et F sont chevauchants, il est avantageux d'éviter que l'extrémité 3' de E soit hybridée trop près de celle de F. Un espacement de 5 nucléotides constitue une marge de sécurité préférée. Dans le cas d'un procédé d'amplification utilisant la transcription, il est indispensable que l'extrémité 5' de l'amorce F contienne une séquence minimum du promoteur incluse dans l'amorce A, cette séquence minimum devant permettre ultérieurement la fixation d'une ARN polymérase et l'initiation de la transcription. La longueur des amorces E et F peut être comprise entre 5 et 100 nucléotides et comprendre une partie de la séquence cible, mais ne dépassant pas la longueur de la région cible comprise entre les amorces A et B.

L'élongation simultanée des amorces E et F sur la matrice ARN, en présence d'une ADN polymérase comprenant notamment une activité ARN dépendante et d'un excès de désoxyribonucléosides triphosphates, conduit au déplacement du brin issu de F. Le brin d'ADN ainsi libéré peut s'hybrider avec l'amorce B. Une ADN polymérase (comprenant notamment une activité ADN et/ou ARN dépendante) peut alors élonger l'extrémité 3' de B sur le brin d'ADN et l'extrémité 3' du brin d'ADN sur l'amorce B. L'ADN double brin ainsi obtenu de taille définie possède alors le promoteur fonctionnel de l'amorce A, ainsi que le promoteur fonctionnel de l'amorce B, ce dernier étant précédé d'une séquence arbitraire.

La matrice double brin ainsi obtenue permet de transcrire des ARNs à partir de chaque promoteur situé à l'extrémité de ce fragment de taille définie, en présence d'un excès de ribonucléosides triphosphates et de l'ARN polymérase correspondant à chaque promoteur. Les ARNs transcrit à partir du promoteur de A peuvent alors s'hybrider avec les amorces C et D. Le cycle issu de l'élongation de C et D peut alors recommencer comme décrit précédemment.

Les figures 4 et 6 décrivent un cas particulier de la méthode d'amplification par "Réaction de Transcription utilisant le Déplacement" dans lequel les amorces A et B diffèrent des amorces A et B du procédé général décrit aux figures 3 et 5.

En effet, les amorces A et B ne contiennent, en plus de la séquence définie arbitraire et de la séquence complémentaire ou homologue de la séquence cible, qu'une partie de la séquence sens d'un promoteur d'une ARN polymérase, cette partie incluant l'extrémité 3' de cette séquence sens d'un promoteur, et ne donnant pas lieu, sous forme double brin, à la génération d'un promoteur fonctionnel, c'est à dire à même d'induire l'initiation de la transcription par une ARN polymérase.

Les amorces C, D, E, F, G et H sont telles que décrites précédemment dans le procédé général. En particulier, les amorces F et D comprennent tout ou partie d'une séquence sens de promoteur.

La figure 4 décrit comme suit une voie d'entrée pour le cycle d'amplification décrit par la figure 6 ; comme dans la description de la figure 3, sur la cible dénaturée ou simple brin s'hybrident les amorces A et G, et éventuellement B et H si la cible est double brin. Les mêmes enzymes sont ajoutées au milieu réactionnel précédemment décrit. L'élongation simultanée de ces amorces par une ADN polymérase conduit au déplacement du brin d'ADN provenant de l'élongation de A ou B par élongation du brin issu de G ou H respectivement. Les ADNs simple brin obtenus peuvent alors s'hybrider avec les amorces B et H, ou A et G respectivement. L'élongation de G ou H provoque le déplacement du produit d'élongation de A ou B respectivement. Les deux simples brins d'ADN de longueur définie ainsi générés, portant une séquence promoteur sens incomplète, peuvent s'hybrider avec les amorces D et C, ou F et E respectivement. Le produit d'élongation de C ou E déplace le produit d'élongation de E ou F respectivement, générant chacun un simple brin d'ADN de longueur définie, et portant une séquence sens de promoteur complète, provenant des amorces D ou F. Ces brins d'ADN peuvent s'hybrider respectivement avec les amorces A ou B, dont l'élongation génère chacune un acide nucléique double brin correspondant à la séquence cible encadrée à une extrémité par un promoteur pour une ARN polymérase entier et fonctionnel, et l'autre extrémité par une partie d'un promoteur non fonctionnel, suivie d'une séquence définie arbitraire. Comme la voie d'entrée décrite à la figure 3, la voie d'entrée décrite à la figure 4 peut être réalisée à partir d'une cible capturée sur un support solide.

Les ADNs double brin ainsi obtenus peuvent rentrer dans le cycle d'amplification décrit par la figure 6. Chacun permet la transcription de multiples copies d'un ARN à partir du promoteur complet fonctionnel, l'un générant un brin comprenant la séquence cible et l'autre générant un brin comprenant la séquence complémentaire de la cible. Ces brins d'ARNs peuvent alors respectivement s'hybrider soit avec les amorces E et F, soit avec les amorces C et D. Comme dans le cycle d'amplification décrit à la figure 5, les amorces C et D, ou E et F peuvent être soit adjacentes, soit contiguës, soit chevauchantes (pourvu que l'hybridation sur l'ARN de l'extrémité 3' de F ou D soit possible). L'élongation simultanée de E et F ou de C et D sur leur matrice ARN respective conduit au déplacement du brin issu de F ou de D respectivement. Les deux brins d'ADN ainsi libérés peuvent s'hybrider respectivement avec B ou A. Deux double brin d'ADN de longueur définie sont générés par élongation des amorces A et B sur les ADNs néosynthétisés et élongation des ADNs néosynthétisés sur ces amorces. Ils peuvent à nouveau rentrer dans le cycle car ils sont identiques aux deux ADNs double brin générés lors de la voie d'entrée.

Comme le cycle décrit à la figure 5, le cycle décrit à la figure 6 provoque une amplification exponentielle d'ARN et d'ADN correspondant aux deux brins complémentaires de la cible initiale.

La nature et la longueur des amorces utilisées dans cette invention, les séquences de promoteurs utilisés et les concentrations d'ARN polymérases relatives à chaque type de promoteur lorsqu'elles sont présentes pourront être choisis de façon à privilégier une voie d'amplification par rapport à l'autre, de manière à obtenir préférentiellement une forme ou une autre d'ADN ou d'ARN. Il est ainsi possible d'obtenir majoritairement un ARN simple brin directement détectable dans les procédés de détection des produits d'amplification, en aval de la présente invention, sans étape de dénaturation des acides nucléiques.

L'ARN issu d'une des deux voies de la méthode d'amplification « DTR » décrite (figure 5) étant substrat pour la deuxième, et *vice et versa,* il apparaît donc que cette méthode est une technique d'amplification cyclique dont l'accumulation des produits de réaction s'effectue de manière exponentielle. Chaque étape de transcription à partir d'un promoteur permet d'obtenir, à partir d'une matrice ADN, entre 500 et 1000 copies d'ARN. Chaque ARN permet d'obtenir une copie d'ADNc qui conduira à une matrice ADN disponible pour la transcription de 500 à 1000 copies d'ARN complémentaire du premier. Il en résulte donc qu'en un seul cycle de ladite méthode d'amplification, on aura une multiplication de la séquence cible par un facteur 2,5 x 10⁵ à 10⁶ . Pour un temps de réaction minimum pour la méthode d'amplification « DTR » (par exemple une heure ou plus), plusieurs cycles de réaction pourront se produire, et ceci jusqu'à l'épuisement des réactifs d'amplification tels que les nucléosides triphosphates et les amorces, conduisant à une amplification dont le rendement correspond à 10⁹ à 10¹² molécules d'ADN et d'ARN produites pour une seule molécule cible initiale. En fonction des concentrations des réactifs utilisés, et notamment des différentes amorces, on peut privilégier, par la méthode d'amplification « DTR », la production de l'une ou l'autre forme des acides nucléiques (ADN et/ou ARN), et l'un ou l'autre des brins de l'acide nucléique cible de départ.

Les termes "fragment d'acide nucléique", "segment d'acide nucléique" ou "oligonucléotide" tels qu'utilisés dans la présente demande, signifient un fragment d'ADN ou d'ARN naturel, un polynucléotide naturel ou de synthèse, un fragment d'ADN ou d'ARN de synthèse non modifié ou comprenant au moins une base modifiée telles que l'inosine, la méthyl-5-désoxcytidine, la diméthylamino-5-désoxyuridine, la désoxyuridine, la diamino-2,6-purine, la bromo-5-désoxyuridine, la pseudouridine, la pseudoisocytidine ou toute autre base modifiée permettant l'hybridation. Ce polynucléotide possède une longueur d'au moins 5 désoxyribonucléotides ou ribonucléotides comprenant éventuellement au moins un nucléotide modifié comme précédemment décrit. Ce polynucléotide peut aussi être modifié au niveau de la liaison internucléotidique (comme par exemple les liaisons phosphorothioate, H-phosphonate, alkyl phosphonate), au niveau du squelette comme par exemple les alpha-oligonucléotides (brevet Français n° 2 607 507) ou les PNA (Egholm *et al.*, 1992. *J. Am. Chem. Soc.* 114 : 1895-1897). Chacune des modifications peut être prise en combinaison.

Le terme "support solide" tel qu'utilisé ici inclut tous les matériaux sur lesquels peut être immobilisé un fragment d'acide nucléique pour une utilisation dans des tests diagnostiques, en chromatographie d'affinité et dans des processus de séparation. Des matériaux naturels, de synthèse, poreux ou non, magnétiques ou non, modifiés ou non chimiquement peuvent être utilisés comme support solide, notamment les polysaccharides tels que les matériaux de cellulose, par exemple du papier, des dérivés de cellulose tels que l'acétate de cellulose et la nitrocellulose ; des polymères tels que le chlorure de vinyle, polyéthylène, polystyrène, polyacrylate ou copolymères tels que polymère de chlorure de vinyle et de propylène, polymère de chlorure de vinyle et acétate de vinyle ; copolymères à base de styrènes ; des fibres naturelles telles que le coton et des fibres synthétiques telles que le nylon ; des céramiques; de la silice. Les supports pouvant être utilisés dans la présente invention sont un polymère de polystyrène, un copolymère butadiène-styrène ou un copolymère butadiène-styrène en mélange avec un ou plusieurs polymères ou copolymères choisis parmi le polystyrène, les copolymères styrène-acrylonitrile ou styrène-méthylmétacrylate de méthyle, les polypropylènes, les polycarbonates ou analogues. Avantageusement, le support pouvant être utilisé dans la présente invention est un polystyrène ou un copolymère à base de styrène comprenant entre 10 et 90 % en poids de motifs de styrène ou de la silice. Les supports solides peuvent être, sans limitation, sous la forme d'une plaque de microtitrage, d'une feuille, d'un cône, d'un tube, d'un puits, de billes ou analogue.

Le terme "amorce" désigne une structure oligonucléotidique simple brin composée d'au moins cinq nucléotides. Ces nucléotides peuvent être des désoxyribonucléotides et/ou des ribonucléotides. Ces nucléotides peuvent être modifiés comme décrit précédemment dans le paragraphe relatif à la description du terme "fragment d'acide nucléique". Les amorces oligonucléotidiques, une fois hybridées sur une séquence d'acide nucléique (ADN, ARN, ou molécule chimère ADN-ARN) substantiellement complémentaire sont substrats des polymérases. L'extrémité 3'OH de ces amorces peut être élongée, en présence de nucléotides adéquats et d'une polymérase, conduisant à la synthèse d'un brin complémentaire à la séquence matrice sur laquelle est hybridée ladite amorce. Une amorce peut également être constituée par hybridation de l'extrémité d'une séquence d'acide nucléique simple brin sur elle-même, conduisant notamment à la formation de structures en épingle à cheveux ou tige-boucle. Les amorces oligonucléotidiques hybridées sur une séquence d'acide nucléique ont la propriété de fixer à leur extrémité 3'OH les polymérases.

Les exemples suivants illustrent l'invention sans toutefois la limiter. A moins qu'elles ne soient spécifiées, toutes les méthodes relatives à la mise en oeuvre des expériences décrites dans les exemples ci-après ont été réalisées conformément à leur description par Sambrook *et al*. (1989. Molecular Cloning : A Laboratory Manual, 2nd Edition, Cold Spring Harbor Laboratory, Cold Spring Harbor).

### EXEMPLE 1 :

La faisabilité de la technique d'amplification d'acides nucléiques par transcription et déplacement, dite "DTR", est démontrée en décomposant les diverses étapes intervenant dans celle-ci. Le principe de cette méthode reposant sur une réaction de transcription utilisant le déplacement, l'étude de la transcription d'une cible ADN après déplacement a été réalisée (figure 2). Le modèle d'étude choisi est la séquence du gène *tem* codant pour la β-lactamase, enzyme conférant une résistance à l'antibiotique ampicilline. La séquence de ce gène est décrite comme la séquence ID n°1. Cette séquence est présente dans le vecteur de clonage pBR322. Cet acide nucléique peut être obtenu par extraction de plasmides d'une culture bactérienne suivie d'une digestion par des endonucléases de restriction, ou être préparé par une technique d'amplification appropriée (Sambrook *et al*. 1989. Molecular Cloning : A Laboratory Manual, 2nd Edition, Cold Spring Harbor Laboratory, Cold Spring Harbor). La faisabilité d'installation d'un promoteur d'ARN polymérase par déplacement de brin, pour l'obtention d'ARNs complémentaires d'une séquence cible (ARN ou ADN) par transcription en phase homogène (figure 2), a été étudiée pour diverses ADN polymérases. Dans ce cas particulier, l'ARN polymérase utilisée est celle du phage T7. Les essais sont réalisés dans un volume final de 50 µl et dans le tampon de réaction 1X décrit par Milligan *et al*. (1987. *Nucl. Acids Res*. 15 : 8783-8798) pour l'utilisation de l'ARN polymérase T7, en présence de dATP, dCTP, dGTP et dTTP (1 mM chaque, Pharmacia), de ATP, CTP, GTP et UTP (4 mM chaque, Boehringer), de 1 U/µl de RNA guard (Pharmacia). La quantité de cible initiale utilisée est de 10¹¹ copies par essai, la concentration de l'ARN polymérase T7 (New England Biolabs) est 1 U/µl et celle de l'ADN polymérase est 0,1 U/µl. L'amorce promotrice A24 (SEQ ID n° : 2) contenant une séquence consensus du promoteur du phage T7 juxtaposée avec une séquence complémentaire de la cible, ainsi que l'amorce Z appelée 1028 (SEQ ID n° : 3), lorsque présentes, sont à une concentration finale de 500 nM. L'amorce de déplacement Y appelée DIS1 (SEQ ID n° : 4) est à une concentration variable allant de 0 à 5 µM. Les différents réactifs, excepté le mélange enzymatique susceptible d'être inactivé par la chaleur (ADN polymérase, ARN polymérase et RNA guard) sont mis en présence de la cible et dénaturés 3 minutes à 95°C (cible initiale constituée ADN double brin) ou 65°C (cible initiale constitué d'ARN simple brin), puis refroidis sur glace pour l'addition du mélange enzymatique. La concentration en glycérol, due à l'addition des enzymes, est égale à 5 %. Une incubation de 2 heures à 37°C est alors réalisée, avant que la réaction ne soit arrêtée par congélation à -20°C.

Une fraction du mélange réactionnel (0,2 volume, soit 10 µl) est analysée par électrophorèse en gel de polyacrylamide dénaturant, préparé selon la méthode décrite par Sambrook *et al*. (1989. Molecular Cloning: A Laboratory Manual, 2nd Edition, Cold Spring Harbor Laboratory, Cold Spring Harbor). Les gels sont formés par polymérisation d'une solution contenant 15 % d'acrylamide, urée 7 M, 0,26 % bisacrylamide, 0,6 ‰ persulfate d'ammonium, 90 mM Tris-borate, 2 mM EDTA, pH 8,3, en présence de 1 ‰ TEMED. Ils sont coulés dans des appareils d'électrophorèse "mini-protean II electrophoresis cell" (Biorad) et ont une épaisseur de 1 mm. Les échantillons à analyser sont préparés par mélange de 10 µl de mélange réactionnel et de 10 µl d'une solution 0,035 % xylène cyanol, 0,035 % bleu de bromophenol, 1 mM EDTA, 98 % formamide. Avant dépôt sur gel, ces échantillons sont dénaturés à 65°C pendant 2 minutes, puis refroidis rapidement sur glace. L'électrophorèse est effectuée à 150 Volts, jusqu'à ce que le bleu de bromophénol migre à 1 cm du bas du gel. Les gels sont alors colorés pendant 10 minutes dans une solution de bromure d'ethidium à 0,6 µg/ml, et photographiés sur une table UV (312 nm) grâce à un appareil MP4 (Polaroïd).

Les produits séparés par électrophorèse sont transférés sur membrane de nylon Hybond N. (Amersham) grâce à un appareil "mini trans-blot electrophoretic transfert cell" (Biorad), dans un tampon 45 mM Tris-Borate, 1 mM EDTA, pH 8,3, à 4°C, sous un champ électrique de 35 Volts . heure⁻¹ . cm⁻¹ . Les membranes sont ensuite séchées durant 5 minutes à 80°C, puis les acides nucléiques sont fixés sur la membrane par exposition aux rayonnements UV (312 nm) durant 3 minutes.

Les membranes sont préhybridées par incubation à 37°C pendant 60 minutes dans 4 ml de tampon phosphate de sodium 0,1 M pH 7,0, chlorure de sodium 0,5 M, EDTA 1 mM, SDS 0,65 %, ADN de saumon 0,14 mg/ml, et polyéthylène glycol 6000 (PEG) 2 %. L'hybridation est effectuée par incubation durant 60 minutes à 37°C dans 5 ml du même tampon contenant, à la concentration de 200 ng/ml l'oligonucléotide A28 (SEQ ID n° : 5), complémentaire du produit de transcription attendu (correspondant au brin de la séquence ID n° : 1) du gène *tem*, et marqué à la peroxydase de raifort par couplage en 5' selon le procédé précédemment décrit dans le brevet International WO 91/19812. Après 3 lavages de 30 secondes dans 50 ml de tampon PBS 1X (Sambrook *et al.*, 1989. Molecular Cloning : A Laboratory Manual, 2nd Edition, Cold Spring Harbor Laboratory, Cold Spring Harbor) contenant 0,5 ‰ de Tween 20, l'oligonucléotide hybridé est révélé par l'activité de la peroxydase en présence de 10 mg de tétrahydrochlorure de diaminobenzidine dihydraté (DAB) dans 20 ml de tampon phosphate de sodium 20 mM pH 7,2, chlorure de sodium 150 mM, albumine de sérum de bovin 2 mg/ml. Après incubation à température ambiante et à l'abri de la lumière pendant 15 minutes, la réaction est arrêtée par rinçage à l'eau distillée.

Des essais de transcription dépendante du déplacement ont été réalisés à partir d'une cible constituée du plasmide pBR322 préalablement clivé par l'endonucléase *Alu* I puis purifié (10¹¹ copies/essai). L'ADN polymérase utilisée est une ADN polymérase ADN-dépendante, le fragment de Klenow (Boehringer, 5 U/essai), en absence ou en présence de différentes concentrations d'amorce de déplacement Y appelée DIS1 (SEQ ID n° : 4), à savoir : 5 µM (environ 10¹⁴ copies), 500 nM, 50 nM, 5 nM ou 0,5 nM. Un témoin d'activité de l'ARN polymérase T7 a été préalablement constitué en préparant un fragment d'ADN double brin par PCR, à partir de la cible pBR322 clivée par *Alu* I, et à l'aide des amorces A24 (SEQ ID n° : 2) et 1028 (SEQ ID n° : 3). Bien entendu, les fragments double brin à extrémités définies comprenant au moins un promoteur, pouvant être préparés par exemple par clonage d'une séquence dans un plasmide contenant un promoteur, digestion par des enzymes de restriction et purification sur gel d'agarose suivie d'une électroélution. Le produit de 285 paires de bases ainsi obtenu contient alors à une extrémité le promoteur du phage T7 et sa transcription par l'ARN polymérase T7 permet d'obtenir un ARN de 263 bases correspondant au produit de réaction attendu lors des essais de transcription dépendante du déplacement. Le témoin d'activité de l'ARN polymérase T7 est réalisé par incubation de 10¹¹ copies du fragment de 285 paires de bases dans les conditions précédemment décrites, mais en absence d'ADN polymérase et de dNTPs. Cette réaction est effectuée pendant deux heures, en parallèle des essais de déplacement.

La figure 8 illustre les résultats obtenus lors des essais de déplacement de brin par le fragment de Klenow, en présence de quantités variables d'amorce de déplacement Y. Comme attendu, les résultats montrent que la présence d'un produit de transcription est limitée aux échantillons contenant une amorce de déplacement Y. Les résultats de transcription positifs en présence d'amorce de déplacement Y mettent en évidence la capacité de déplacement de brin du fragment de Klenow. En effet, la présence d'ARNs complémentaires de la cible *tem* et de taille correspondant à l'espacement des amorces X et Z sur la cible *tem*, c'est à dire identiques à ceux issus du témoin de transcription, provient de la transcription à partir d'un fragment d'ADN double brin identique à celui du témoin de transcription par l'ARN polymérase T7 et contenant, comme ce dernier, un promoteur d'ARN polymérase fonctionnel (double brin). Ce fragment ne peut être obtenu, dans les conditions de réaction décrites, que par élongation de l'amorce Z hybridée sur le produit d'élongation de l'amorce promotrice X (figure 2) contenant le promoteur T7. Or, l'hybridation et l'élongation de l'amorce Z n'est possible qu'en présence d'un produit d'élongation de l'amorce promotrice X sous forme simple brin. La présence de ce produit d'élongation sous forme simple brin ne peut provenir que de l'élongation de l'amorce de déplacement Y qui provoque la libération du produit d'élongation de l'amorce promotrice X.

Les résultats montrent également que l'intensité du produit de transcription final varie en fonction de la concentration de l'amorce de déplacement Y dans les essais. La quantité de matériel transcrit étant proportionnelle à la quantité de matrice disponible pour l'ARN polymérase T7, il apparaît donc que la quantité de matrice synthétisée par déplacement et élongation diffère en fonction de la quantité d'amorce de déplacement Y utilisée dans un essai. La concentration d'amorce Z étant constante et en excès, la quantité de matrice double brin disponible dépend de celle du produit simple brin d'élongation de l'amorce promotrice. Il s'avère donc que la quantité de produit d'élongation libéré (simple brin) varie en fonction de la concentration de l'amorce de déplacement dans les essais. Dans les conditions utilisées, une concentration d'amorce de déplacement de 50 nM (soit 10 fois la quantité de cible présente), correspondant à un ratio amorce de déplacement / amorce promotrice égal à 1/10 donne le meilleur rendement de déplacement et donc de transcription. Ces résultats montrent l'influence du ratio amorce de déplacement / amorce promotrice pour le rendement de déplacement d'un brin d'acide nucléique par élongation d'une amorce située en amont de ce dernier. De part et d'autre de cet optimum, le rendement de déplacement diminue sensiblement : si la concentration d'amorce de déplacement augmente, les conditions thermodynamiques favorisent l'hybridation de celle-ci sur la cible et donc son élongation, au détriment de l'hybridation puis l'élongation de l'amorce promotrice ; de même, les résultats montrent que si la concentration d'amorce de déplacement diminue, la probabilité d'hybridation de celle-ci en amont de l'amorce promotrice diminue, et par voie de conséquence, la probabilité d'élongation pour le déplacement du brin situé en aval diminue.

Cet exemple démontre la possibilité d'utiliser les propriétés de déplacement de brin d'une polymérase pour l'installation d'une séquence promotrice sur une séquence cible donnée. Cette méthode a pour avantage de permettre l'obtention d'une matrice ADN pour l'ARN polymérase, sans recourir à une étape de dénaturation thermique, chimique ou nécessiter l'action d'une activité enzymatique de type nucléase afin de libérer le produit d'élongation d'une amorce promotrice sur un acide nucléique simple brin. Par le biais d'une étape de transcription associée, il est donc possible de synthétiser un grand nombre de copies d'ARN correspondant à la séquence cible comprise entre les deux amorces X et Z. La figure 8 montre que la séquence cible ne peut être détectée par la méthode utilisée sans étape d'amplification par transcription (piste 2). En revanche, l'utilisation d'une ADN polymérase douée d'activité de déplacement de brin, associée à une amorce de déplacement, permet d'obtenir une matrice permettant la synthèse de multiples brins d'ARN correspondant à la cible, et dont le nombre permet leur détection, indiquant la présence de la cible dans l'échantillon initial. Cet exemple démontre donc l'intérêt de la méthode afin d'installer un promoteur d'ARN polymérase par déplacement de brin pour l'obtention de multiples brins d'ARN complémentaires d'une séquence cible par transcription en phase homogène (une seule étape et une seule température). Cette méthode permet d'obtenir directement des transcrits d'une séquence cible par addition d'un mélange réactionnel comprenant une amorce Y, une amorce X, une amorce Z, des désoxyribonucléosides triphosphates et des ribonucléosides triphosphates, une ADN polymérase douée d'une capacité de déplacement de brin et une ARN polymérase, à une cible d'acide nucléique préalablement dénaturée, notamment dans le cas d'une cible double brin. La méthode est donc composée d'une étape unique, sans addition ultérieure ou intermédiaire de réactifs, ni utilisation d'activité enzymatique, en particulier d'activité nucléase.

### EXEMPLE 2 :

La faisabilité de la méthode "DTR" implique également que la transcription en phase homogène, comme décrite dans l'exemple 1, puisse également être réalisée à partir d'une molécule ARN. Cette molécule d'ARN peut être soit la cible initiale dont la méthode « DTR » permet de déterminer la présence par amplification, ou un produit intermédiaire du cycle de la méthode d'amplification "DTR" (figure 5). Ce produit intermédiaire peut être, à titre d'exemple, l'ARN obtenu par transcription dans le cas de l'exemple 1. Cette faisabilité suppose l'utilisation d'une ADN polymérase à la fois ARN- et ADN-dépendante qui possède une capacité de déplacement de brin. Afin de démontrer cette faisabilité, des ARNs, c'est à dire correspondant à la séquence complémentaire de la séquence de *tem* (SEQ ID n° : 1), ont été synthétisés in vitro à l'aide de l'ARN polymérase T7, selon les conditions réactionnelles du kit MEGAscript (Ambion), à partir de 10¹² copies de matrice ADN. Pour ce faire, une matrice d'ADN double brin a été synthétisée par PCR, à partir de pBR322, et à l'aide des amorces PROANTI (SEQ ID n° : 6) et ORF1 (SEQ ID n° : 7). Le produit de 889 paires de bases ainsi obtenu contient alors à une extrémité le promoteur du phage T7 et sa transcription par l'ARN polymérase T7 permet d'obtenir un ARN de 867 bases correspondant à la séquence complémentaire de la séquence du gène *tem* (SEQ ID n° : 1). Les ARNs synthétisés sont traités à la DNase I afin d'éliminer l'ADN matrice, purifiés par extraction au phénol/chloroforme et précipités à l'éthanol en présence de sels d'acétate d'ammonium. Les ARNs ainsi obtenus sont repris dans de l'eau préalablement traitée au diéthylpyrocarbonate (DEPC), analysés par électrophorèse en gel de polyacrylamide dénaturant et dosés par absorbance à 260 nm. Une quantité équivalente à 10¹¹ copies d'ARN cible (5 nM final) est utilisée par essai et les conditions de réaction sont identiques à celles décrites dans l'exemple 1, en présence des amorces X, Y et Z précédemment décrites. Cependant, l'ADN polymérase utilisée dans le cadre de ces essais est la transcriptase inverse du virus AMV (Seikagaku) et le ratio amorce de déplacement/amorce promotrice fixé à l'optimum précédemment déterminé (1/10) a été utilisé, ce qui correspond à une concentration finale de l'amorce de déplacement égale à 50 nM final. L'analyse des produits de réaction est réalisée comme décrit dans l'exemple 1, par séparation électrophorétique, transfert sur membrane et hybridation à l'aide de la sonde A28 (SEQ ID n° : 5) marquée à la peroxydase de raifort. La figure 9 illustre les résultats obtenus lors des différents essais réactionnels. Une quantité équivalente à 10¹² copies du fragment de PCR de 285 paires de bases, contenant un promoteur T7 à une extrémité, obtenu à l'aide des amorces A24 (SEQ ID n° : 2) et 1028 (SEQ ID n° : 3), a été déposée (piste 1) afin de servir de marqueur de taille pour l'ARN attendu (263 bases), correspondant à la transcription d'un fragment identique, lors des essais réalisés. Les essais ont été réalisés en absence de transcriptase inverse et ARN polymérase T7 (piste 2), et en présence des enzymes, avec les amorces X, Y et Z (piste 3), sans amorce de déplacement Y (piste 4) ou sans amorce Z (piste 5). L'ARN de 263 bases attendu n'est détecté qu'à partir de l'essai comportant l'ensemble des réactifs enzymatiques et des amorces, tandis qu'en absence d'amorce de déplacement, aucun produit de transcription n'est obtenu. Ce résultat confirme donc la faisabilité du déplacement de brin par l'action d'une ADN polymérase et démontre la faisabilité du déplacement sur matrice ARN, grâce à la capacité de déplacement de brin de la transcriptase inverse AMV. Cette dernière est donc capable, lors de l'élongation d'une amorce située en amont d'une amorce précédemment élongée, de dissocier un hétéroduplex ARN-ADN. La stabilité de ce dernier étant connue pour être supérieure à celle d'un homoduplex ADN-ADN, il est *a fortiori* probable que cette transcriptase inverse soit capable, par élongation d'amorce, de déplacer un brin d'ADN hybridé sur une cible ADN. Bien qu'une activité RNase H associée à la transcriptase inverse AMV soit décrite, aucun produit de transcription n'est obtenu en l'absence d'amorce de déplacement. Ceci suggère que les conditions de tampon utilisées dans ces essais ne sont pas propices à cette activité RNase H ou que la séquence de la cible ARN utilisée est peu sensible à l'action de cette dernière. L'ARN du duplex ARN cible-ADNc ne peut donc être dégradé et le produit d'élongation de l'amorce promotrice ne peut donc être libéré pour l'hybridation et l'élongation de l'amorce Z sur ce dernier. Ce résultat démontre donc, outre la capacité de déplacement de brin de la transcriptase inverse AMV, le manque total d'efficacité, dans ces conditions, de la séparation d'un ADNc d'un hétéroduplex ADNc-ARN par action de la RNase H associée à la transcriptase inverse AMV, justifiant l'utilisation de RNase H exogène d'*Escherichia coli* dans les méthodes d'amplification transcriptionnelles précédemment décrites, comme la NASBA, la 3SR ou la LAT. En absence d'amorce Z (mais en présence d'amorce de déplacement Y), aucun produit de transcription n'est obtenu, ce qui montre qu'aucune réaction d'autoamorçage ne se produit à l'extrémité 3' de l'ADNc issu de l'élongation de l'amorce promotrice, dans les conditions de la présente réaction, bien que cette propriété ait été décrite chez les transcriptases inverses dans des conditions particulières.

Cet exemple démontre donc l'efficacité de la méthode pour installer un promoteur d'ARN polymérase par déplacement de brin pour l'obtention d'ARNs complémentaires d'une séquence cible d'ARN, par transcription en phase homogène (une seule étape et une seule température) tel que décrit dans la figure 2, et pour produire de multiples brins d'ARN complémentaires d'un brin d'ARN cible donné. La méthode est donc composée d'une étape unique, sans addition ultérieure ou intermédiaire de réactifs, et ne requiert aucune activité nucléase, en particulier RNase H, pour la séparation d'un ADNc d'un hétéroduplex ARN-ADN. En particulier, dans les conditions de réaction, la transcriptase inverse AMV ne montre pas d'activité RNase H qui permettrait la libération, en absence d'amorce de déplacement, du brin élongé à partir de l'amorce promotrice sur matrice ARN. La capacité de déplacement de brin de la transcriptase inverse AMV démontre donc la faisabilité du cycle d'amplification utilisant la transcription et le déplacement et par conséquent le caractère exponentiel de l'accumulation des produits de réaction à partir d'une cible ARN ou ADN (figure 5).

### EXEMPLE 3 :

Afin de confirmer la faisabilité de la séparation d'une molécule d'ADN d'un hétéroduplex ADN-ARN par déplacement de brin, et ce indépendamment de toute activité RNase H résiduelle intrinsèque à certaines transcriptases inverses, des essais ont été réalisés en utilisant une transcriptase inverse MMLV dépourvue d'activité RNase H. Une telle enzyme, obtenue par génie génétique, est disponible commercialement sous l'appellation "Superscript^{II}"® (GIBCO-BRL). Les essais ont été réalisés comme dans l'exemple 2, à partir de la même cible ARN, à l'aide des amorces X, Y et Z précédemment décrites. La « Superscript^{II} »® est ajoutée à une concentration finale de 4 U/µl (200 U/essai) et les amorces de déplacement et promotrices sont présentes à un ratio de 1/10. Les produits de réaction sont analysés de la façon décrite dans l'exemple 1. Les résultats obtenus sont présentés dans la figure 10. Il apparaît que la présence d'un produit de transcription est limitée à l'échantillon comportant une amorce de déplacement Y (piste 7), ce qui confirme la capacité de déplacement de brin de la transcriptase inverse MMLV dépourvue d'activité RNase H. En absence d'amorce de déplacement (piste 6), aucun signal de transcription n'est obtenu, confirmant la validité de la séparation d'un ADN d'un hétéroduplex ARN-ADN par les propriétés de déplacement de brin des transcriptases inverses. En absence d'amorce Z (piste 5), aucun produit de transcription n'est obtenu, ce qui confirme, comme dans l'exemple 2, que la transcriptase inverse MMLV "Superscript^{II}"® ne réalise pas d'autoréamorçage de l'extrémité 3' de l'ADNc dans ces conditions de réaction.

Cet exemple confirme donc la faisabilité de la méthode d'amplification par transcription utilisant le déplacement, à partir d'une cible ARN, et ce de manière totalement indépendante des activités RNase H éventuellement présentes dans le milieu réactionnel. Cette méthode d'amplification peut donc être réalisée à l'aide d'une transcriptase inverse et d'une ARN polymérase, sans aucune autre activité enzymatique supplémentaire.

### EXEMPLE 4 :

La faisabilité de la méthode d'amplification "DTR" repose également sur la capacité d'ARN polymérases à transcrire des ARNs complémentaires à partir d'une seule et même matrice double brin comportant des promoteurs d'ARN polymérase fonctionnels à chacune de ses extrémités, en particulier dans la méthode décrite aux figures 3 et 5. Afin de valider cette hypothèse, une matrice ADN double brin a été synthétisée par PCR, en présence des amorces DTA1 (SEQ ID n° : 8) et DTA2 (SEQ ID n° : 9), à partir de la cible pBR322. Le fragment de 275 paires de bases ainsi obtenu est purifié. Il contient une partie de la séquence du gène *tem* (du nucléotide 312 à 486 inclus de la SEQ ID n° : 1), encadré par deux promoteurs de l'ARN polymérase du phage T7, orientés pour la transcription de l'un ou l'autre des brins de la séquence *tem*. Chaque séquence sens promotrice est précédée en 5' par une séquence correspondant à l'amorce F220 (SEQ ID n° : 10). La transcription d'un tel fragment à partir de chacun des promoteurs situés à ses extrémités doit théoriquement conduire à la synthèse de deux populations d'ARN complémentaires l'un de l'autre et comportant à leur extrémité 3' la séquence antisens du promoteur T7 (c'est-à-dire la séquence complémentaire de la séquence sens du promoteur T7) suivie de la séquence complémentaire de l'oligonucléotide F220 (SEQ ID n° : 10). La taille des ARNs attendus est de 231 bases, et ils contiennent la séquence comprise entre les nucléotides 312 à 486 (inclus) du gène *tem* (SEQ ID n° : 1), ou la séquence complémentaire de celle-ci. Des essais de transcription sont réalisés, à partir du fragment de PCR portant un promoteur à chaque extrémité, dans le tampon 1X décrit par Milligan *et al*. (1987. *Nucl. Acids Res*. 15 : 8783-8798), dans un volume final de 50 µl, en présence d'ATP, CTP, GTP et UTP (4 mM chaque), de 1 U/µl d'ARN polymérase du phage T7 (New England Biolabs) et 1 U/µl de RNA guard (Pharmacia). La matrice PCR est utilisée à 10¹¹ copies par essai et la concentration en glycérol due à l'addition des enzymes est de 5 %. La transcription est effectuée pendant 2 heures à 37°C et arrêtée par congélation à -20°C. Le produit de réaction (10 µl) est analysé par séparation électrophorétique en gel de polyacrylamide dénaturant, puis transféré sur membrane de Nylon, comme décrit dans l'exemple 1. Deux membranes sont ainsi préparées afin de réaliser une hybridation de la sonde A28 (SEQ ID n° : 5) marquée en 5' par la peroxydase de raifort, d'une part, et de la sonde A19 (SEQ ID n° : 11) également marquée en 5' par la peroxydase de raifort, d'autre part. La sonde A19 permet de détecter les ARNs, complémentaires de la séquence de *tem* décrite (SEQ ID n° : 1), tandis que la sonde A28 permet la détection des ARNs correspondant à la séquence de *tem* décrite (SEQ ID n° : 1). La figure 11 illustre les résultats obtenus lors de ces essais. Les sondes A28 et A19 permettent de détecter 10¹² copies de la matrice d'ADN cible double brin de 275 paires de bases (piste 1), ainsi que des ARNs de 231 bases dans le cas des essais transcriptionnels (piste 2). La détection des transcrits attendus à l'aide des deux types de sondes A19 (partie A) et A28 (partie B) montre que la transcription d'ARNs complémentaires a été réalisée par l'ARN polymérase T7, à partir de chaque promoteur de la matrice ADN. L'obtention d'ARNs complémentaires à partir d'une matrice ADN commune contenant un promoteur à chacune de ses extrémités démontre que l'ARN polymérase T7 est capable de réaliser la polymérisation d'ARNs par progression convergente sur une même matrice. La possibilité d'obtention d'ARN complémentaires dans ces conditions à l'aide d'une ARN polymérase confirme donc la faisabilité de la méthode d'amplification d'acides nucléiques « DTR » décrite aux figures 3 et 5. Ces résultats confirment donc que le cycle d'amplification décrit aux figures 3 et 5 possède un caractère exponentiel, conduisant à une importante accumulation d'ARN et d'ADN dont la séquence correspond à la partie de la cible comprise entre les amorces promotrices A et B (figure 5).

### EXEMPLE 5 :

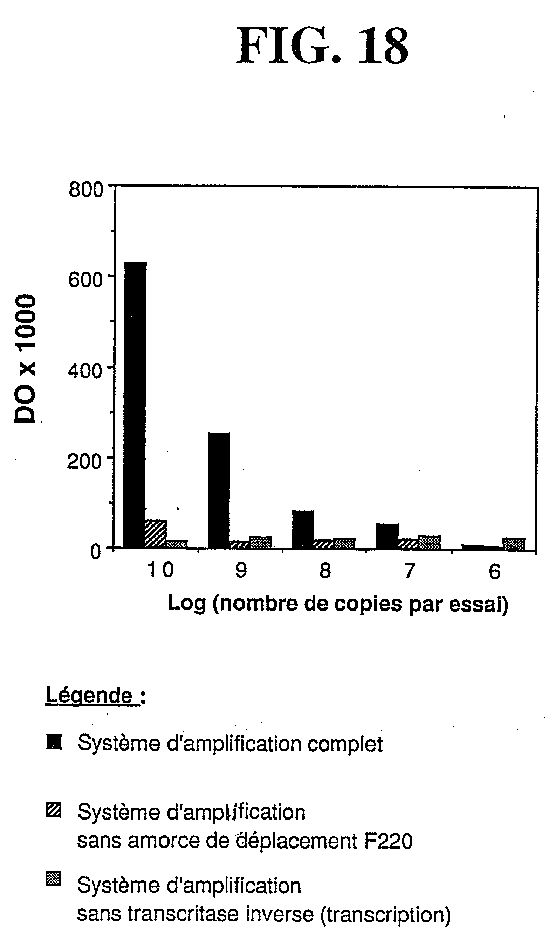

La validité expérimentale de la méthode d'amplification "DTR" a été prouvée par l'étude d'un des cycles d'amplification décrits dans la présente demande, et en particulier tel que décrit dans la figure 15. Pour ce faire un fragment de PCR de 167 paires de bases a été synthétisé à l'aide des amorces DTA7 (SEQ ID n° : 12) et DTA8 (SEQ ID n° : 13), à partir de pBR322, et qui contient donc une partie de la séquence du gène *tem* (du nucléotide 336 à 402 inclus de la SEQ ID n° : 1). Ce fragment de PCR correspond à un type de molécule d'entrée dans le cycle d'amplification et contient par conséquent à chacune de ses extrémités une séquence promotrice d'ARN polymérase (en particulier le promoteur du phage T7), précédée en 5' d'une séquence définie telle que la séquence F220 (SEQ ID n° : 10). Afin de mettre en évidence le caractère exponentiel de la méthode, différentes réactions d'amplification ont été menées en parallèle en présence de quantités décroissantes de molécules cibles constituées par le fragment de PCR précédent purifié (correspondant à l'entrée dans le cycle d'amplification) : de 10¹⁰ à 10⁶ copies par essai. Les réactions ont été réalisées dans un volume final de 50 µl, en présence d'ATP, CTP, GTP et UTP (4 mM chaque), de dATP, dCTP, dGTP et dTTP (1 mM chaque) de 1 U/µl d'ARN polymérase du phage T7 (New England Biolabs), de 4 U/µl de Transcriptase inverse MMLV "Superscript^{II}"® (GIBCO-BRL), dépourvue d'activité RNase H, et 1 U/µl de RNA "Guard"® (Pharmacia). Le milieu réactionnel contenait en outre les différentes amorces promotrices A et B correspondant à DTA7 et DTA8 (SEQ ID n° : 12 et SEQ ID n° : 13, respectivement) à une concentration de 0,01 µM et l'amorce de déplacement C équivalente à E correspondant à F220 (SEQ ID n° : 10) à une concentration de 1 µM. Après deux heures d'incubation à 37°C, les réactions d'amplification sont arrêtées par congélation du milieu réactionnel à -20°C. Une fraction de 5 µl, soit 1/10 de volume réactionnel est analysée quantitativement par capture et détection spécifique selon la méthode ELOSA (Enzyme Linked Oligo Sorbent Assay). La méthode fait intervenir la fixation d'un oligonucléotide de capture sur support solide (plaque de microtitrage), la dénaturation du produit de réaction, son hybridation sur la sonde de capture spécifique de la séquence amplifiée et la révélation par une sonde de détection couplée à la peroxydase de raifort. L'oligonucléotide de capture A20 (SEQ ID n° : 14) est fixé de façon passive sur les puits d'une plaque de microtitrage "Nunc-Immuno plate"® de Maxisorp selon la méthode déjà décrite dans le brevet FR 91 09057, permettant la fixation d'environ 5 pmoles d'oligonucléotide sur un puits. Après fixation, trois lavages avec du tampon PBS Tween 1X sont effectués. La détection du produit d'amplification capturé est réalisée conformément à la technique précédemment décrite dans le brevet FR 91 09057. La fraction de 5 µl à analyser est ajoutée à un volume de 35 µl de tampon phosphate de sodium 0,2 M pH 7,0, chlorure de sodium 1 M, EDTA 2 mM, SDS 1,3%, ADN de saumon 0,24 mg/ml, polyéthylène glycol (PEG) 6000 4%. Les acides nucléiques contenus dans cet échantillon sont dénaturés par addition de 5 µl d'hydroxyde de sodium 2 M à température ambiante, et neutralisés après 3 minutes par addition de 5 µl d'acide acétique 2 M. Pour contrôle et étalonnage, deux gammes de dilution d'ARN correspondant aux produits d'amplification attendus sont effectuées. Les échantillons sont alors déposés dans le puits d'une plaque de microtitrage dans lequel a été préalablement fixée la sonde oligonucléotidique de capture A20 (SEQ ID n° : 14). On dépose soit 50 µl d'échantillon non dilué, soit le même volume d'échantillon dilué au 10^{ème} ou au 100^{ème}. Immédiatement, un volume de 50 µl de tampon phosphate de sodium 0,2 M pH 7,0, chlorure de sodium 1 M, EDTA 2 mM, SDS 1,3%, ADN de saumon 0,24 mg/ml, polyéthylène glycol (PEG) 6000 4%, contenant 5 ng de sonde oligonucléotidique de détection A18 (SEQ ID n° : 15) couplée à la peroxydase de raifort, est ajouté. Après une incubation de 60 minutes à 37°C, les puits sont lavés au PBS Tween 1X. La révélation de la sonde A18-peroxydase hybridée sur le produit d'amplification est effectuée par addition de 100 µl d'une solution contenant le substrat ortho-phénylène diamine (OPD). La réaction colorimétrique est arrêtée après 20 minutes par addition de 100 µl d'acide sulfurique 1 M. La densité optique à 492 nm est lue grâce à un lecteur "AXIA microreader"® (bioMérieux). Les résultats obtenus sont représentés par les histogrammes dans la figure 18. Pour chacune des dilutions de cible, trois essais ont été réalisés : essai complet (comme décrit précédemment), essai sans amorce de déplacement F220 (SEQ ID n° : 10) et essai sans transcriptase inverse MMLV "Superscript^{II}"® Les résultats montrent que la méthode d'amplification (système complet) permet de détecter dans ces conditions un signal spécifique significatif jusqu'à une quantité de cible initiale de 10⁷ copies par essai, soit une sensibilité de 10⁶ copies, puisqu'une fraction équivalant à 1/10^{ème} du milieu réactionnel est analysée dans ces conditions. Cette sensibilité n'est que relative et peut être grandement améliorée si l'on utilise un système de révélation autre que la colorimétrie (par exemple la fluorescence, la chemiluminescence ou la bioluminescence). Les résultats montrent surtout qu'en absence d'amorce de déplacement F220, un signal spécifique n'est obtenu que pour une quantité de cible égale 10¹⁰ copies par essai. La différence de sensibilité de détection entre "avec" et "sans" amorce de déplacement est donc d'un facteur 10³, ou de 3 unités Log de base 10. Ceci démontre que la méthode permet une accumulation importante de produit d'amplification, celle-ci ne pouvant provenir que de la réalisation du cycle d'amplification. Cette cyclisation n'est donc possible que par la présence d'une amorce de déplacement telle que l'amorce F220. De même, si l'on supprime la transcriptase inverse MMLV "Superscript^{II}"®, on ne peut obtenir un signal de détection que pour une quantité de cible initiale supérieure ou égale à 10¹⁰ copies par essai. Ces dernières conditions de réaction correspondent en fait à une étape de transcription réalisée sur la molécule bifonctionnelle correspondant à l'entrée dans le cycle d'amplification, comme décrit dans l'exemple 4. Ces données montrent donc que la méthode est extrêmement plus sensible que la transcription seule et que cette sensibilité repose sur la cyclisation du procédé jusqu'à l'étape de transcription par le biais d'une étape de déplacement enzymatique, en particulier à l'aide d'une amorce de déplacement en présence d'une ADN polymérase telle qu'une transcriptase inverse. L'analyse qualitative des essais d'amplification par séparation électrophorétique, transfert sur membrane de nylon et hybridation, comme décrit dans l'exemple 1, à l'aide de la sonde A28 (SEQ ID n° : 5) d'une part et de la sonde A18 (SEQ ID n° : 15) d'autre part couplées à la peroxydase de raifort, a permis de mettre en évidence deux molécules d'ARN complémentaires de 120 bases, correspondant au produit d'amplification attendu (117 bases).

De la même façon, si l'on réalise les essais d'amplification précédents dans un milieu contenant, conformément à la figure 5, outre la première amorce de déplacement C (équivalente à E, dans ce cas particulier) correspondant à F220 (SEQ ID n° : 10), une deuxième amorce de déplacement D (équivalente à F) correspondant à T7pro (SEQ ID n° : 16), on obtient des résultats et une sensibilité similaires à ceux obtenus avec un seul type d'amorce de déplacement. Ceci montre que la technique peut être simplifiée et est suffisamment évolutive pour être adaptée en fonction des cas particuliers liés à la nature de la cible nucléique à amplifier.

Le seuil de détection de la méthode de détection colorimétrique utilisée dans cet exemple, supérieur ou égal 10¹⁰ copies par essai, permet donc de déduire un facteur d'amplification supérieur ou égal à 10⁴ en deux heures de réaction.

### EXEMPLE 6 :

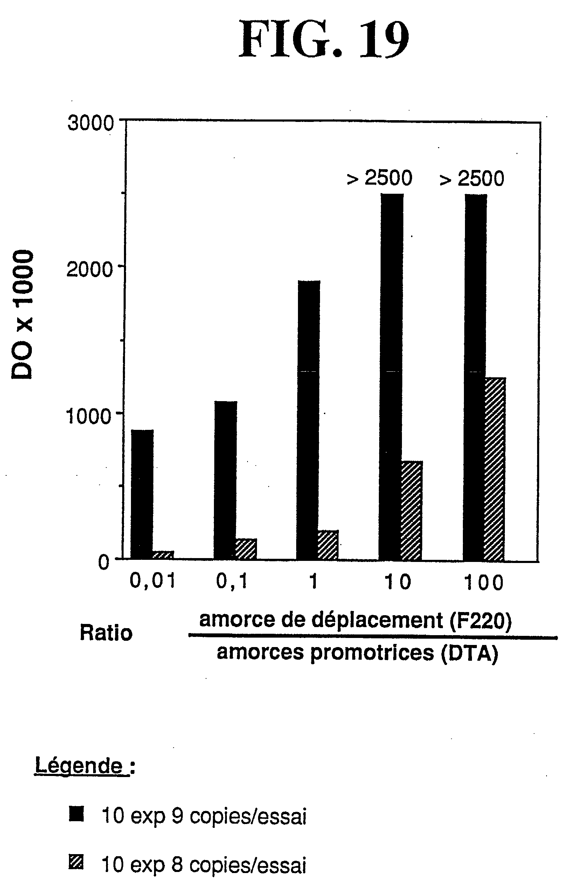

La méthode d'amplification "DTR" décrite dans la présente demande repose sur un cycle exponentiel utilisant de manière combinée la transcription et le déplacement de brin. En particulier, le déplacement de brin enzymatique fait intervenir, tel que décrit dans la figure 15, une transcriptase inverse, une amorce de déplacement C (équivalente à E dans cet exemple) et deux amorces promotrices (A et B) devant être déplacées. Au sein du cycle d'amplification, le ratio amorce de déplacement/amorce déplacée (ici amorce promotrice) influence de manière importante le rendement d'amplification de la méthode. Si on réalise l'amplification dans les conditions réactionnelles précédentes, en présence de 10⁸ ou 10⁹ copies de cible initiale, avec une concentration fixe d'amorces promotrices A et B correspondant à DTA7 (SEQ ID n° : 12) et DTA8 (SEQ ID n° : 13) égale à 0,1 µM, et une concentration d'amorce de déplacement C correspondant à F220 (SEQ ID n° : 10) égale à 0,001, 0,01, 0,1, 1 ou 10 µM, on obtient un rendement d'amplification croissant avec le ratio amorce de déplacement/amorce déplacée (figure 19). L'analyse quantitative sur plaque de microtitrage, comme décrit dans l'exemple 5, en utilisant une sonde de capture A25 (SEQ ID n° : 17) et une sonde de détection A28 (SEQ ID n° : 5), montre (figure 19) que le ratio amorce de déplacement/amorce déplacée égal à 100 est le plus favorable dans ce cas (on notera qu'une valeur égale à 2500 correspond à une saturation optique de l'appareil et correspond donc à un signal supérieur ou égal à 2500). Ce ratio doit être adapté selon la longueur des amorces de déplacement ou promotrices utilisées, ou selon la nature de la séquence cible, afin de favoriser l'étape de cyclisation par déplacement enzymatique de brin .

### EXEMPLE 7 :

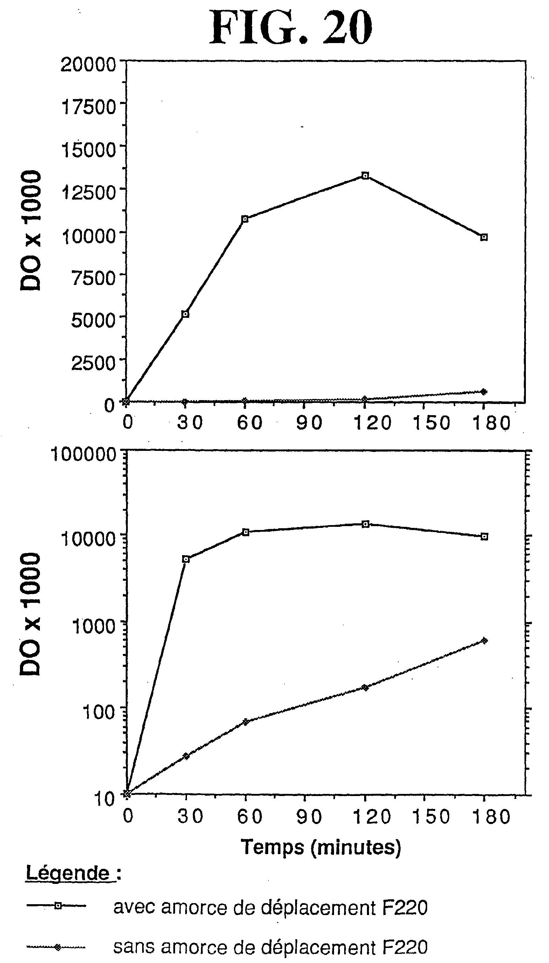

L'efficacité de l'amplification exponentielle de la technique "DTR" peut être mise en évidence par une analyse de la cinétique d'apparition du signal d'amplification. Pour ce faire, une réaction d'amplification a été réalisée à partir de 10⁹ copies de molécule cible, conformément aux conditions de l'exemple 5. A des intervalles réguliers, une fraction de 5 µl de mélange réactionnel a été prélevée et congelée immédiatement à -20°C. L'analyse quantitative du signal d'amplification de ces fractions a été réalisée comme décrit dans l'exemple 5, en utilisant une sonde de capture A25 (SEQ ID n° : 17) et une sonde de détection A28 (SEQ ID n° : 5). En parallèle, un essai d'amplification a été réalisé en absence d'amorce de déplacement F220. Les résultats indiqués par les courbes de la figure 20 montrent clairement que l'apparition du signal est exponentielle et très rapide, puisqu'un plateau est atteint dès 1 heure de réaction. En parallèle, la réaction d'amplification en absence d'amorce de déplacement, correspondant donc à une simple réaction de transcription, conduit à une augmentation linéaire du signal. La méthode d'amplification "DTR", par opposition à la transcription seule, est donc une méthode conduisant à l'accumulation rapide et exponentielle d'un produit à partir d'une séquence cible initiale.

### EXEMPLE 8 :

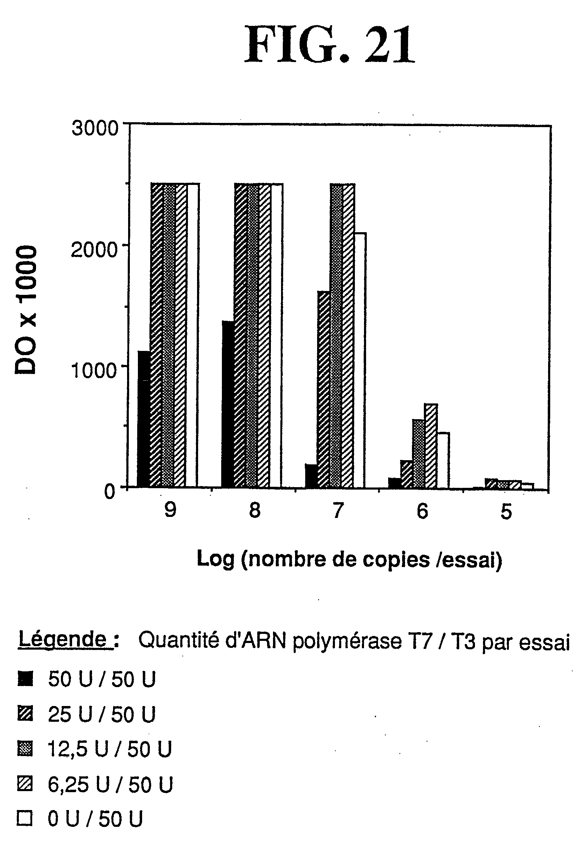

La méthode d'amplification "DTR" décrite dans la présente demande conduit essentiellement à un ARN double brin correspondant à une partie de la séquence cible. Afin de faciliter la détection de cet ARN double brin par les méthodes d'hybridation, ou dans des cas particuliers de production d'ARN simple brin, il peut être intéressant de favoriser l'apparition d'un brin d'ARN au détriment de son complémentaire. Ceci peut être réalisé par l'utilisation combinée de deux types d'amorces promotrices A et B comprenant un promoteur différent d'ARN polymérase phagique (par exemple T7 et T3), en présence de concentrations d'ARN polymérases déséquilibrées. Par exemple, nous avons synthétisé par PCR, une molécule d'ADN double brin de 167 paires de bases correspondant à l'entrée dans l'un des cycles d'amplification de la méthode "DTR" (figure 15) en utilisant l'amorce DTA8 (SEQ ID n° : 13) comprenant un promoteur T7 et DTA9 (SEQ ID n° : 18) contenant un promoteur T3. Des essais d'amplification ont été réalisés sur des dilutions de cette cible, conformément à l'exemple 5, en présence de concentrations d'ARN polymérase T7 variant de 0 à 50 U/essai et une concentration d'ARN polymérase T3 constante égale à 50 U/essai. La figure 21 montre que l'utilisation du ratio d'enzyme 6,25 U d'ARN polymérase T7 pour 50 U d'ARN polymérase T3 permet d'obtenir, par capture et détection à l'aide des sondes A25 (SEQ ID n° : 17) et A28 (SEQ ID n° : 5) respectivement, un signal d'amplification accru et conduit à un signal significatif jusqu'à une quantité de cible de 10⁵ copies par essai, ce qui correspond à une sensibilité de 10⁴ copies de molécules cibles, par révélation colorimétrique. Le facteur multiplicatif de la méthode d'amplification de cible "DTR" sachant que la sensibilité de la méthode de détection est de 10¹⁰ copies, est donc de 10⁵; ce qui confère à cette méthode une performance la rendant utile dans de nombreuses applications.

LISTE DES SEQUENCES

SEQ ID n° : 1 :
ATGAGTATTC AACATTTCCG TGTCGCCCTT ATTCCCTTTT TTGCGGCATT 50
TTGCCTTCCT GTTTTTGCTC ACCCAGAAAC GCTGGTGAAA GTAAAAGATG 100
CTGAAGATCA GTTGGGTGCA CGAGTGGGTT ACATCGAACT GGATCTCAAC 150
AGCGGTAAGA TCCTTGAGAG TTTTCGCCCC GAAGAACGTT TTCCAATGAT 200
GAGCACTTTT AAAGTTCTGC TATGTGGCGC GGTATTATCC CGTGTTGACG 250
CCGGGCAAGA GCAACTCGGT CGCCGCATAC ACTATTCTCA GAATGACTTG 300
GTTGAGTACT CACCAGTCAC AGAAAAGCAT CTTACGGATG GCATGACAGT 350
AAGAGAATTA TGCAGTGCTG CCATAACCAT GAGTGATAAC ACTGCGGCCA 400
ACTTACTTCT GACAACGATC GGAGGACCGA AGGAGCTAAC CGCTTTTTTG 450
CACAACATGG GGGATCATGT AACTCGCCTT GATCGTTGGG AACCGGAGCT 500
GAATGAAGCC ATACCAAACG ACGAGCGTGA CACCACGATG CCTGCAGCAA 550
TGGCAACAAC GTTGCGCAAA CTATTAACTG GCGAACTACT TACTCTAGCT 600
TCCCGGCAAC AATTAATAGA CTGGATGGAG GCGGATAAAG TTGCAGGACC 650
ACTTCTGCGC TCGGCCCTTC CGGCTGGCTG GTTTATTGCT GATAAATCTG 700
GAGCCGGTGA GCGTGGGTCT CGCGGTATCA TTGCAGCACT GGGGCCAGAT 750
GGTAAGCCCT CCCGTATCGT AGTTATCTAC ACGACGGGGA GTCAGGCAAC 800
TATGGATGAA CGAAATAGAC AGATCGCTGA GATAGGTGCC TCACTGATTA 850
AGCATTGGTA A 861

SEQ ID n° : 2 :
AATTCTAATA CGACTCACTA TAGGGAGACC CCGAAGAACG TTTTC 45

SEQ ID n° : 3 :
CTCCTTCGGT CCTCCGATC 19

SEQ ID n° : 4 :
AGTTGGGTGC ACGAGTGGG 19

SEQ ID n° : 5 :
CACTCATGGT TATGGCA 17

SEQ ID n° : 6 :
AATTCTAATA CGACTCACTA TAGGGAGATT ACCAATGCTT AATCA 45

SEQ ID n° : 7 :
ATGAGTATTC AACATTTC 18

SEQ ID n° : 8 :
TCTAATCCTG TTTGCTCCCC AGTAATTTAA TACGACTCAC TATAGAAAGA 50
ACCAGTCACA GAAAGGCAT 69

SEQ ID n° : 9 :
TCTAATCCTG TTTGCTCCCC AGTAATTTAA TACGACTCAC TATAGAAAGA 50
ACGATCAAGG CGAGTTACA 69

SEQ ID n° : 10 :
TCTAATCCTG TTTGCTCCCC 20

SEQ ID n° : 11 :
TGCCATAACC ATGAGGT 17

SEQ ID n° : 12 :
TCTAATCCTG TTTGCTCCCC AGTAATTTAA TACGACTCAC TATAGAAAGA 50
GGATGGCATG ACAGTA 66

SEQ ID n° : 13 :
TCTAATCCTG TTTGCTCCCC AGTAATTTAA TACGACTCAC TATAGAAAGA 50
GTTGGCCGCA GTGTTAT 67

SEQ ID n° : 14 :
AGAGAATTAT GCAGTGC 17

SEQ ID n° : 15 :
ATAACACTGC GGCCAAC 17

SEQ ID n° : 16 :
TAATACGACT CACTATAG 17

SEQ ID n° : 17 :
GCACTGCATA ATTCTCT 17

SEQ ID n° : 18 :
TCTAATCCTG TTTGCTCCCC AGTAATTAAT TAACCCTCAC TAAAGGGAAC 50
GGATGGCATG ACAGTA 66

## Revendications

1. Méthode pour générer un polynucléotide double brin contenant une séquence complémentaire d'une séquence d'intérêt et un polynucléotide simple brin contenant une séquence complémentaire de ladite séquence d'intérêt, ladite méthode comprenant les étapes consistant :
- à obtenir un acide nucléique simple brin comprenant ladite séquence d'intérêt, et
- à mettre en contact ledit acide nucléique simple brin avec une première et une seconde amorces, en présence d'une activité de polymérase et d'une activité de déplacement de brin, ladite première amorce s'hybridant à une première séquence dudit acide nucléique simple brin et ladite seconde amorce s'hybridant à une seconde séquence dudit acide nucléique simple brin, ladite seconde séquence chevauchant ladite première séquence, et lesdites première et seconde séquences étant situées en aval de l'extrémité 3' de ladite séquence d'intérêt, de sorte que le produit d'élongation de l'une des amorces déplace le produit d'élongation de l'autre amorce, grâce à quoi on obtient un polynucléotide double brin contenant une séquence complémentaire de ladite séquence d'intérêt et un polynucléotide simple brin déplacé contenant une séquence complémentaire de ladite séquence d'intérêt.

2. Méthode selon la revendication 1, dans laquelle l'une desdites première et seconde amorces est présente en excès par rapport à l'autre amorce.

3. Méthode selon l'une quelconque des revendications précédentes, dans laquelle ledit acide nucléique est de l'ADN.

4. Méthode selon la revendication 1 ou 2, dans laquelle ledit acide nucléique est de l'ARN.

5. Méthode selon l'une quelconque des revendications précédentes, dans laquelle l'activité de polymérase est une ADN polymérase.

6. Méthode selon l'une quelconque des revendications précédentes, dans laquelle l'activité de déplacement de brin est apportée par une ADN polymérase.
